# EUROPÄISCHE PATENTSCHRIFT

(11) **EP 0 601 416 B1**
(45) Veröffentlichungstag und Bekanntmachung des Hinweises auf die Patenterteilung: **15.07.1998**
(21) Anmeldenummer: 93119096.1
(22) Anmeldetag: 26.11.1993
(51) Int. Cl.: C09B 62/445, C09B 62/06, C07C 261/04, D06P 3/66, D06P 1/382, D06P 1/384

(54) **Wasserlösliche Anthrachinonverbindungen, Verfahren zu ihrer Herstellung und ihre Verwendung als Farbstoffe**
Watersoluble anthraquinone compounds, process for their preparation and their use as dyestuffs
Composés d'anthraquinone solubles dans l'eau, leur procédé de préparation et leur utilisation comme colorants

(30) Priorität: 05.12.1992 DE 4241035; 22.06.1993 DE 4320562
(43) Veröffentlichungstag der Anmeldung: 15.06.1994
(73) Patentinhaber: DyStar Textilfarben GmbH & Co. Deutschland KG, 60318 Frankfurt am Main (DE)
(72) Erfinder: Dannheim, Jörg, Dr., D-60489 Frankfurt am Main (DE); Hähnle, Reinhard, Dr., D-61462 Königstein/Ts. (DE); Russ, Werner Hubert, Dr., D-65439 Flörsheim/Main (DE)

(56) Entgegenhaltungen:
- EP-A- 0 024 676
- EP-A- 0 087 394
- DATABASE WPI Week 8303, Derwent Publications Ltd., London, GB; AN 83-06389K[03] & JP-A-57 199 878 (SUMITOMO CHEMICAL K. K.) 7. Dezember 1982 & JP-A-57 199 878 (...)
- PATENT ABSTRACTS OF JAPAN vol. 12, no. 301 (C-521)(3148) 16. August 1988 & JP-A-63 072 668 (TEIJIN LTD) 2. April 1988

## Beschreibung

Die Erfindung liegt auf dem technischen Gebiet der faserreaktiven Farbstoffe.

Aus der japanischen Patentanmeldungs-Veröffentlichung Sho-57-199 878 sind unter anderem anthrachinoide Farbstoffe bekannt, die Cyanamido-, N-Cyanobenzamido- und N-Cyanosulfonamido-Gruppen enthalten. Sie besitzen jedoch teilweise anwendungstechnische Mängel, wie niedrige Fixiergrade, niedrige Farbstärke und fehlende Brillanz.

Mit der vorliegenden Erfindung wurden nunmehr Anthrachinonverbindungen gefunden, die sich als faserreaktive Farbstoffe vorteilhaft gegenüber den bekannten Farbstoffen unterscheiden und Färbungen mit hoher Farbstärke in brillanten blauen Tönen liefern. Die neuen Anthrachinonverbindungen entsprechen der allgemeinen Formel (1) 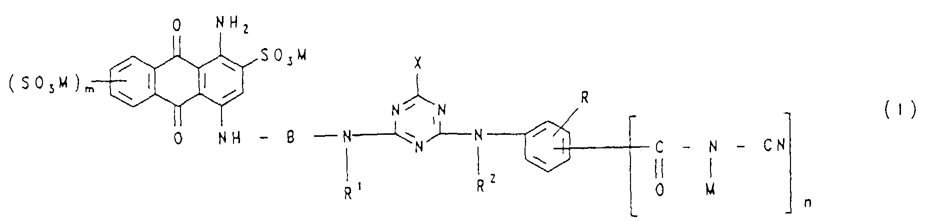 in welcher bedeuten:
- m: ist die Zahl Null, 1 oder 2 (wobei im Falle von m gleich Null diese Gruppe für Wasserstoff steht);
- B: ist Phenylen, das durch 1 bis 2 Sulfogruppen und 1 bis 3 Alkylreste von 1 bis 4 C-Atomen, wie Ethyl und insbesondere Methyl, substituiert ist; oder ist Phenylen-alkylen oder Alkylen-phenylen, wobei deren Alkylenreste solche von 1 bis 4 C-Atomen sind und die Phenylenreste durch 1, 2 oder 3 Substituenten aus der Gruppe Alkyl von 1 bis 4 C-Atomen, wie Ethyl und insbesondere Methyl, und Sulfo substituiert sind; oder ist (C₅-C₈)-Cycloalkylen oder Alkylen-(C₅-C₈)-cycloalkylen oder (C₅-C₈)-Cycloalkylenalkylen oder Alkylen-(C₅-C₈)-cycloalkylen-alkylen, wobei die Cycloalkylenreste, wie Cyclohexylenreste, noch durch 1 oder 2 Methylgruppen substituiert sein können und die Alkylenreste solche von 1 bis 4 C-Atomen sind; oder ein Rest der allgemeinen Formel -phen-D-phen-ist, in welcher jedes phen eine zueinander gleiche oder voneinander verschiedene Bedeutung besitzt und einen Phenylenrest, vorzugsweise para-Phenylenrest, bedeutet, der durch 1 oder 2 Substituenten aus der Gruppe Sulfo, Alkyl C₁₋₄ und Alkoxy C₁₋₄ substituiert ist, und D eine direkte Bindung oder eine Gruppe der Formel -NH-, -O-, -SO₂-, -CONH-, -NHCO-, -SO₂NH-, -NHSO₂- und -SO₂NHSO₂- bedeutet,
- R¹: ist Wasserstoff oder Alkyl von 1 bis 4 C-Atomen, wie Methyl und Ethyl, bevorzugt Wasserstoff;
- R²: ist Wasserstoff oder Alkyl von 1 bis 4 C-Atomen, wie Methyl und Ethyl, bevorzugt Wasserstoff;
- X: ist Fluor oder Chlor;
- R: ist Wasserstoff, Alkyl von 1 bis 4 C-Atomen, wie Ethyl und insbesondere Methyl, oder Alkoxy von 1 bis 4 C-Atomen, wie Ethoxy und insbesondere Methoxy;
- n: ist die Zahl 1 oder 2; und
- M: ist Wasserstoff oder ein Alkalimetall, wie Natrium, Kalium und Lithium, oder das Moläquivalent eines Erdalkalimetalls, wie des Calciums.

Bevorzugte Reste der allgemeinen Formel (2) 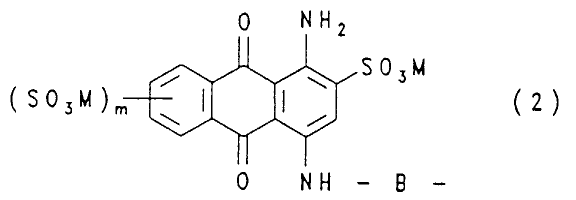 sind beispielsweise 1-Amino-2-sulfo-anthrachinon-4-amino-(3'-sulfo-2',4',6'-trimethyl)-phen-5'-yl, 1-Amino-2-sulfo-anthrachinon-4-amino-(2'-sulfo)-phenyl-4'-methyl, 1-Amino-2-sulfo-anthrachinon-4-amino-cyclohex-4'-yl, 1-Amino-2-sulfo-anthrachinon-4-amino-(4'-methyl)-cyclohex-3'-yl, 1-Amino-2-sulfo-anthrachinon-4-amino-(2'-sulfo)-phenyl-5'-methyl, 1-Amino-2-sulfo-anthrachinon-4-amino-(3'-sulfo-2',6'-dimethyl)-phenyl-5'-methyl, 1-Amino-2-sulfo-anthrachinon-4-amino-(2'-sulfo-4'-methyl)-phenyl-6'-methyl, 1-Amino-2,6-disulfo-anthrachinon-4-amino-(2'-sulfo-4'-methyl)-phenyl-6'-methyl, 1-Amino-2-sulfo-anthrachinon-4-amino-(2'-sulfo-6'-methyl)-phen-4'-yl, 1-Amino-2-sulfo-anthrachinon-4-amino-(4'-sulfo-6'-methyl)-phen-3'-yl, 1-Amino-2-sulfo-anthrachinon-4-amino-(3'-sulfo-2',4',6'-trimethyl)-phenyl-5'-methyl, 1-Amino-2,6-disulfo-anthrachinon-4-amino-(3'-sulfo-6'-methyl)-phen-5'-yl, 1-Amino-2-sulfo-anthrachinon-4-amino-(3'-sulfo-6'-methyl)-phen-5'-yl, 1-Amino-2,5-disulfo-anthrachinon-4-amino-(6'-methyl)-phen-5'-yl, 1-Amino-2,6-disulfo-anthrachinon-4-amino-(6'-methyl)-phen-5'-yl, 1-Amino-2,6-disulfo-anthrachinon-4-amino-(3'-sulfo-2',6'-dimethyl)-phenyl-5'-methyl, 1-Amino-2,7-disulfo-anthrachinon-4-amino-(3'-sulfo-2',6'-dimethyl)-phenyl-5'-methyl, 1-Amino-2,5-disulfo-anthrachinon-4-amino-(2'-sulfo-4'-methyl)-phenyl-3'-methyl, 1-Amino-2,8-disulfo-anthrachinon-4-amino-(4'-methyl)-phenyl-3'-methyl, 1-Amino-2,5,8-trisulfo-anthrachinon-4-amino-(2',6'-dimethyl)-phenyl-3'-methyl, 1-Amino-2,8-disulfo-anthrachinon-4-amino-(2'-sulfo)-phenyl-3'-methyl, 1-Amino-2,5-disulfo-anthrachinon-4-amino-(3'-sulfo-6'-methyl)-phen-5'-yl, 1-Amino-2,7-disulfo-anthrachinon-4-amino-eth-2'-yl, 1-Amino-2-sulfo-anthrachinon-4-amino-phen-3'-yl-1"-sulfonylamidosulfonyl-4"-sulfo-phen-3"-yl, 1-Amino-2,6-disulfo-anthrachinon-4-amino-phen-4'-yl-1"-sulfonylamidosulfonyl-phen-4"-yl und 1-Amino-2-sulfo-anthrachinon-4-amino-phen-3'-yl-1"-sulfonylamidosulfonyl-phen-3"-yl, vorzugsweise 1-Amino-2-sulfo-anthrachinon-4-amino-(2',4',6'-trimethyl-5'-sulfo)-phen-3-yl.

Von den erfindungsgemäßen Anthrachinonverbindungen der allgemeinen Formel (1) können insbesondere diejenigen hervorgehoben werden, die der allgemeinen Formel (3a) und (3b) 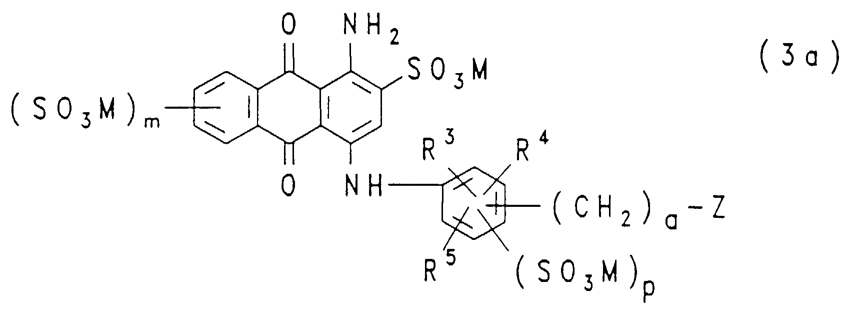 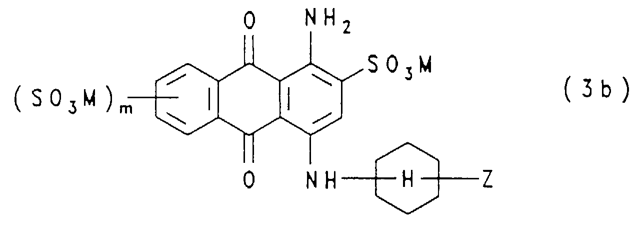 entsprechen. In diesen Formeln bedeuten:
- M und m: haben eine der obengenannten, insbesondere bevorzugten Bedeutungen;
- R³: ist Alkyl von 1 bis 4 C-Atomen, wie Ethyl und insbesondere Methyl;
- R⁴: ist Wasserstoff oder Alkyl von 1 bis 4 C-Atomen, wie Ethyl und insbesondere Methyl;
- R⁵: ist Wasserstoff oder Alkyl von 1 bis 4 C-Atomen, wie Ethyl und insbesondere Methyl;
- p: ist die Zahl 1 oder 2, bevorzugt 1;
- a: ist die Zahl Null oder 1, bevorzugt Null, und
- Z: ist ein Rest der allgemeinen Formel (4) 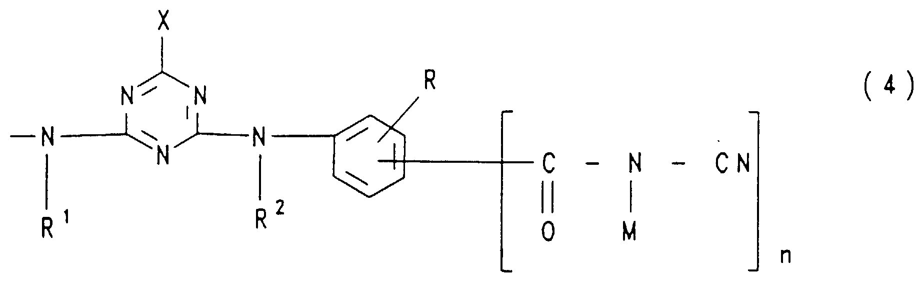 in welcher R¹, R², R, M, n und X die obengenannten, insbesondere bevorzugten, Bedeutungen haben und X für Chlor oder Fluor und hiervon insbesondere bevorzugt für Chlor steht.

In der allgemeinen Formel (3a) sind die Formelreste R³, R⁴ und R⁵ jeweils bevorzugt Methylgruppen.

Insbesondere bevorzugt von diesen hervorgehobenen Anthrachinonverbindungen sind solche Anthrachinonverbindungen, die der allgemeinen Formel (5) 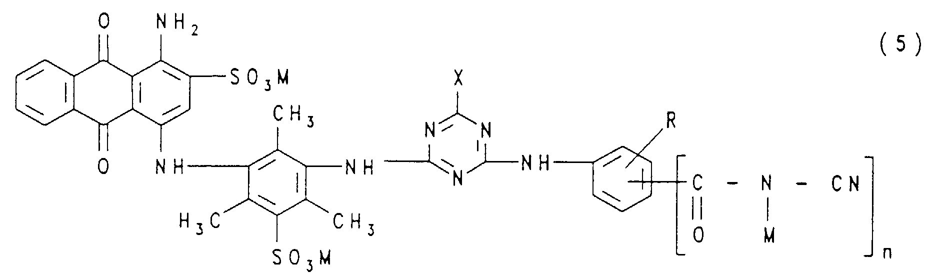 entsprechen, in welcher M, R und n eine der obengenannten, insbesondere bevorzugten, Bedeutungen haben, X für Chlor oder Fluor und bevorzugt Chlor steht, n bevorzugt die Zahl 1 ist und die Cyanamidocarbonyl-Gruppe in meta- oder para-Stellung zur Gruppe -NH- an den Benzolkern gebunden ist.

Die vorliegende Erfindung betrifft ein weiterhin ein Verfahren zur Herstellung der erfindungsgemäßen Anthrachinonverbindungen der allgemeinen Formel (1), bei welchem man eine Halogen-s-triazin-Verbindung entsprechend der allgemeinen Formel (6) 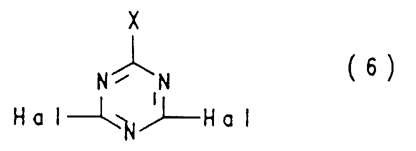 in welcher X eine der obengenannten, insbesondere bevorzugten, Bedeutungen besitzt und Hal für Halogen, wie Chlor oder Fluor, steht, wie beispielsweise 2,4,6-Trifluor-1,3,5-triazin (Cyanurfluorid) oder 2,4,6-Trichlor-1,3,5-triazin (Cyanurchlorid), mit einer aminogruppenhaltigen Anthrachinonverbindung entsprechend der allgemeinen Formel A-NHR¹, worin A eine Verbindung der Formel 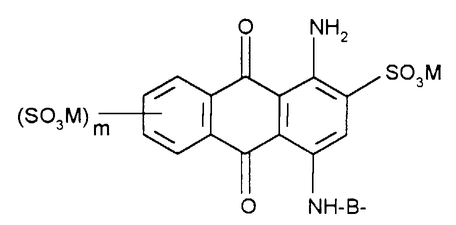 ist und mit einer Aminoverbindung der allgemeinen Formel (7) 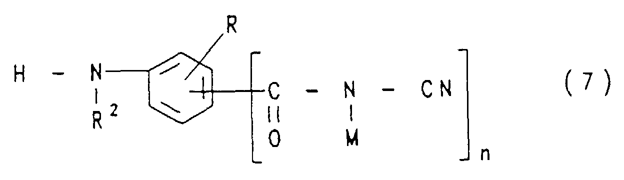 in welcher R², R, M und n eine der obengenannten Bedeutungen haben, in beliebiger Reihenfolge miteinander umsetzt.

Erfindungsgemäße Verfahrensvarianten dieser erfindungsgemäßen Verfahrensweise sind beispielsweise dadurch gekennzeichnet, daß man eine Verbindung der allgemeinen Formel (8) 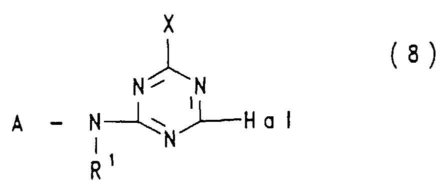 in welcher A, R¹, X und Hal die obengenannten Bedeutungen haben, mit einer Aminoverbindung der allgemeinen Formel (7) umsetzt, oder daß man eine Verbindung der allgemeinen Formel (9) 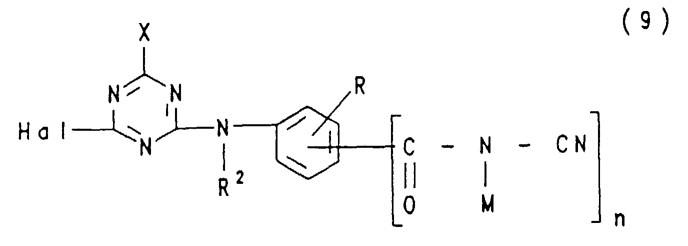 in welcher Hal, X, R², R, M und n die obengenannten Bedeutungen haben, mit einer Verbindung der allgemeinen Formel A-NHR¹ mit A und R¹ der obengenannten Bedeutung umsetzt. Hiervon ist die Verfahrensweise der Umsetzung einer Verbindung der allgemeinen Formel (8) mit einer Verbindung der allgemeinen Formel (7) bevorzugt.

Die Umsetzungen der Ausgangsverbindungen erfolgen im wäßrigen oder wäßrig-organischen Medium in Suspension oder Lösung. Führt man die Umsetzungen in einem wäßrig-organischen Medium durch, so ist das organische Medium beispielsweise Aceton, Dimethylformamid, Dimethylsulfoxid oder N-Methyl-pyrrolidon. Vorteilhaft wird der bei der Kondensation freiwerdende Halogenwasserstoff laufend durch Zugabe wäßriger Alkalihydroxide, -carbonate oder -bicarbonate neutralisiert. Die erste Kondensationsreaktion des Halogen-s-triazins der allgemeinen Formel (6) erfolgt in der Regel bei einer Temperatur zwischen -5°C und +20°C, im Falle von Hal gleich Fluor bevorzugt bei einer Temperatur zwischen -5°C und +5°C, und bei einem pH-Wert zwischen 2 und 10, bevorzugt zwischen 4 und 6. Die nachfolgende Kondensationsreaktion mit der zweiten Aminoverbindung erfolgt in der Regel bei einer Temperatur zwischen 5 und 40°C, im Falle von Hal gleich Fluor bevorzugt bei einer Temperatur zwischen 0 und 20°C, und bei einem pH-Wert zwischen 3 und 9, bevorzugt zwischen 6 und 7.

Insbesondere erfolgt die Umsetzung zwischen einer Verbindung der allgemeinen Formel (8) und einer Verbindung der allgemeinen Formel (7) bei einer Temperatur zwischen 5 und 40°C, bevorzugt zwischen 10 und 30°C, und bei einem pH-Wert zwischen 3 und 9, bevorzugt zwischen 6 und 7, wobei man im Falle, daß in der Verbindung für Formel (8) Hal ein Fluoratom ist, bevorzugt die Umsetzung bei einer Temperatur zwischen 0 und 20°C durchführt.

Ebenfalls erfolgt die Umsetzung einer Verbindung der allgemeinen Formel (9) mit einer Verbindung der allgemeinen Formel A-NHR¹ bei einer Temperatur zwischen 5 und 40°C, bevorzugt zwischen 10 und 30°C, und bei einem pH-Wert zwischen 3 und 9, bevorzugt zwischen 4 und 6, wobei im Falle des Einsatzes einer Verbindung der allgemeinen Formel (9) mit Hal gleich Fluor bevorzugt die Umsetzung bei einer Temperatur zwischen 0 und 20°C durchgeführt wird.

Die Ausgangsverbindungen der allgemeinen Formel (8) lassen sich in an und für sich bekannter Verfahrensweise der Umsetzung von halogensubstituierten Triazinen mit aminogruppenhaltigen Verbindungen herstellen, wie dies beispielsweise oben für die erfindungsgemäßen Verfahrensweisen beschrieben ist, so ebenfalls in wäßrigem oder wäßrig-organischem Medium in der Regel bei einer Temperatur zwischen -5°C und +40°C, bevorzugt zwischen 0 und 30°C, und bei einem pH-Wert zwischen 2 und 10, bevorzugt zwischen 5 und 7, wobei man im Falle einer Ausgangsverbindung der Formel (6) mit Hal gleich Fluor bevorzugt eine Reaktionstemperatur zwischen -5°C und +5°C wählt. In gleicher Weise und unter den gleichen Verfahrensbedingungen lassen sich die Ausgangsverbindungen der allgemeinen Formel (9) durch Umsetzung einer Verbindung der allgemeinen Formel (6) mit einer Verbindung der allgemeinen Formel (7) herstellen.

Die Ausgangsverbindungen der allgemeinen Formel (6) und der allgemeinen Formel A-NHR¹ sind allgemein bekannt und in der Literatur zahlreich beschrieben. Verbindungen der allgemeinen Formel (6) sind beispielsweise Cyanurchlorid und Cyanurfluorid.

Ausgangsverbindungen der allgemeinen Formel A-NHR¹ sind beispielsweise
1-Amino-2-sulfo-4-(3'-sulfo-2',4',6'trimethyl-5'-amino-phenyl)-amino-anthrachinon,
1-Amino-2-sulfo-4-[3',2"-disulfo-4"-amino-diphenyl(4',1")]-amino-anthrachinon,
1 Amino-2-sulfo-4-[3"-sulfo-4"-amino-diphenyl (4',1")]-amino-anthrachinon,
1-Amino-2-sulfo-4-[2'-sulfo-4'-(N-methylamino)-methyl-phenyl]-amino-anthrachinon,
1-Amino-2-sulfo-4-(4'-amino-cyclohexyl)-amino-anthrachinon,
1-Amino-2,6-disulfo-4-(4'-amino-cyclohexyl)-amino-anthrachinon,
1-Amino-2,7-disulfo-4-(4'-amino-cyclohexyl)-amino-anthrachinon,
1-Amino-2,5-disulfo-4-(4'-amino-cyclohexyl)-amino-anthrachinon,
1-Amino-2,8-disulfo-4-(4'-amino-cyclohexyl)-amino-anthrachinon,
1-Amino-2,7-disulfo-4-(4'-methylamino-cyclohexyl)-amino-anthrachinon,
1-Amino-2,6-disulfo-4-(3'-amino-cyclohexyl)-amino-anthrachinon,
1-Amino-2,7-disulfo-4-(3'-amino-cyclohexyl)-amino-anthrachinon,
1-Amino-2-sulfo-4-(4'-methyl-3'-amino-cyclohexyl)-amino-anthrachinon,
1-Amino-2-sulfo-4-[2'-sulfo-4'-(N-methylamino)-methyl-phenyl]-amino-anthrachinon,
1-Amino-2-sulfo-4-[2'-sulfo-5'-(N-methylamino)-methyl-phenyl]-amino-anthrachinon,
1-Amino-2-sulfo-4-(3'-sulfo-2',6'-dimethyl-5'-aminomethyl-phenyl)-amino-anthrachinon,
1-Amino-2-sulfo-4-(2'-sulfo-4'-methyl-6'-aminomethyl-phenyl)-amino-anthrachinon,
1-Amino-2-sulfo-4-[2'-sulfo-4'-methyl-6'-(N-methylamino)-methyl-phenyl]-amino-anthrachinon,
1-Amino-2,6-disulfo-4-[2'-sulfo-4'-methyl-6'-aminomethyl-phenyl]-amino-anthrachinon,
1-Amino-2-sulfo-4-(2'-sulfo-6'-methyl-4'-amino-phenyl)-amino-anthrachinon,
1-Amino-2-sulfo-4-(4'-sulfo-6'-methyl-3'-amino-phenyl)-amino-anthrachinon,
1-Amino-2-sulfo-4-(3'-sulfo-2',4',6'-trimethyl-5'-aminomethyl-phenyl)-amino-anthrachinon,
1-Amino-2,6-disulfo-4-(3'-sulfo-6'-methyl-5'-amino-phenyl)-amino-anthrachinon,
1-Amino-2-sulfo-4-(3'-sulfo-6'-methyl-5'-amino-phenyl)-amino-anthrachinon,
1-Amino-2,5-disulfo-4-(6'-methyl-5'-amino-phenyl)-amino-anthrachinon,
1-Amino-2,6-disulfo-4-(6'-methyl-5'-amino-phenyl)-amino-anthrachinon,
1-Amino-2,6-disulfo-4-[2'-sulfo-4'-(N-methylamino)-methyl-phenyl]-amino-anthrachinon,
1-Amino-2,7-disulfo-4-[2'-sulfo-4'-(N-methylamino)-methyl-phenyl]-amino-anthrachinon,
1-Amino-2,5,8-trisulfo-4-[4'-(N-methylamino)-methyl-phenyl]-amino-anthrachinon,
1-Amino-2,8-disulfo-4-[4'-(N-methylamino)-methyl-phenyl]-amino-anthrachinon,
1-Amino-2,6-disulfo-4-(3'-sulfo-2',6'-dimethyl-5'-aminomethyl-phenyl)-amino-anthrachinon,
1-Amino-2,6-disulfo-4-[3'-sulfo-2',6'-dimethyl-5'-(N-methylamino)-methyl-phenyl]-amino-anthrachinon,
1-Amino-2,7-disulfo-4-(3'-sulfo-2',6'-dimethyl-5'-aminomethyl-phenyl)-amino-anthrachinon,
1-Amino-2,7-disulfo-4-[3'-sulfo-2',6'-dimethyl-5'-(N-methylamino)-methyl-phenyl]-amino-anthrachinon,
1-Amino-2,7-disulfo-4-[2',6'-dimethyl-5'-(N-methylamino)-methyl-phenyl]-amino-anthrachinon,
1-Amino-2,5-disulfo-4-(2'-sulfo-4'-methyl-3'-aminomethyl-phenyl)-amino-anthrachinon,
1-Amino-2,8-disulfo-4-(4'-methyl-3'-aminomethyl-phenyl)-amino-anthrachinon,
1-Amino-2,5,8-trisulfo-4-(2',6'-dimethyl-3'-aminomethyl-phenyl)-amino-anthrachinon,
1-Amino-2,5-disulfo-4-(3'-sulfo-6'-methyl-5'-amino-phenyl)-amino-anthrachinon,
1-Amino-2,6-disulfo-4-[β-(N-methylamino)-ethyl]-amino-anthrachinon,
1-Amino-2-sulfo-4-[3'-(4"-sulfo-3"-amino-phenyl)-sulfonylamidosulfonyl-phenyl]-amino-anthrachinon,
1-Amino-2,6-disulfo-4-[4'-(4"-amino-phenyl)-sulfonylamidosulfonyl-phenyl]-amino-anthrachinon und
1-Amino-2-sulfo-4-[3'-(4"-amino-phenyl)-sulfonylamidosulfonyl-phenyl]-amino-anthrachinon.

Die Ausgangsverbindungen der allgemeinen Formel (7) sind teilweise bekannt; die Verbindungen mit R gleich Alkyl oder Alkoxy sind jedoch noch nicht beschrieben. Verbindungen der allgemeinen Formel (7) mit R gleich Alkyl von 1 bis 4 C-Atomen oder Alkoxy von 1 bis 4 C-Atomen sind deshalb ebenfalls Gegenstand der Erfindung.

Die Verbindungen der allgemeinen Formel (7) lassen sich herstellen, indem man von einer Nitroverbindung der allgemeinen Formel (10) 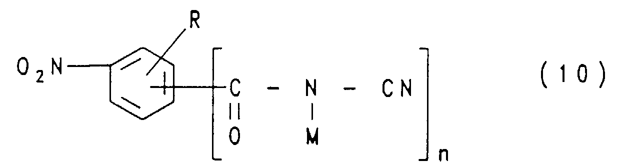 in welcher R, M und n die obengenannten Bedeutungen besitzen, ausgeht und diese in üblicher Weise, bevorzugt katalytisch in wäßriger Lösung oder Suspension, zur Verbindung der allgemeinen Formel (7), in welcher R² gleich Wasserstoff ist, reduziert. Die Einführung einer Alkylgruppe (bei R² gleich Alkyl) kann danach in üblicher Weise analog bekannten Verfahrensweisen mit Hilfe eines Alkylierungsmittels, wie eines Dialkylsulfats, erfolgen.

Die Verbindungen der allgemeinen Formel (10), in welcher R für Alkyl von 1 bis 4 C-Atomen oder Alkoxy von 1 bis 4 C-Atomen steht und n die Zahl 1 oder 2 bedeutet, oder in welcher R Wasserstoff bedeutet und n die Zahl 2 ist, sind ebenfalls noch nicht beschrieben und somit Gegenstand der Erfindung.

Die Ausgangsverbindungen der allgemeinen Formel (10), die, wie erwähnt, teilweise bereits bekannt sind (s. J. Org. Chem. 31, 959-961 (1966) und Chem. Ind. (London) 1963, 1559 + 1560), lassen sich analog diesen literaturbekannten Verfahrensweisen herstellen, so beispielsweise durch Umsetzung der Säurechloride der allgemeinen Formel (11) 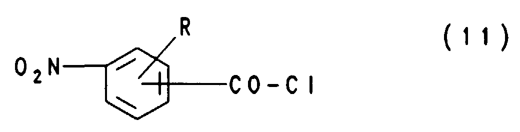 in welcher R die obengenannte Bedeutung besitzt, mit Cyanamid oder einem Salz des Cyanamids in wäßriger Lösung oder Suspension bei einer Temperatur zwischen 0 und 20°C und unter Einhaltung eines pH-Wertes zwischen 7 und 11.

Ausgangsverbindungen der allgemeinen Formel (7) sind beispielsweise 2-Amino-N'-cyano-benzamid, 3-Amino-N'-cyano-benzamid, 4-Amino-N'-cyano-benzamid, 5-Amino-1,3-di-(N'-cyanocarboxamido)-benzol, 3-Amino-4-methoxy-N'-cyano-benzamid und 3-Methyl-4-amino-N'-cyano-benzamid.

Die Abscheidung der erfindungsgemäß hergestellten Farbstoffe der allgemeinen Formel (1), nachfolgend als "Farbstoffe (1)" bezeichnet, aus den Syntheseansätzen erfolgt nach allgemein bekannten Methoden entweder durch Ausfällen aus dem Reaktionsmedium mittels Elektrolyten, wie beispielsweise Natriumchlorid oder Kaliumchlorid, oder durch Eindampfen der Reaktionslösung, beispielsweise durch Sprühtrocknung, wobei dieser Reaktionslösung eine Puffersubstanz zugefügt werden kann. Sie haben faserreaktive Eigenschaften und besitzen sehr gute Farbstoffeigenschaften. Sie können deshalb zum Färben und Bedrucken von hydroxy- und/oder carbonamidgruppenhaltigem Material, insbesondere Fasermaterial, wie auch von Leder, verwendet werden. Ebenso können auch die bei der Synthese der erfindungsgemäßen Verbindungen anfallenden Lösungen, gegebenenfalls nach Zusatz einer Puffersubstanz, gegebenenfalls nach Konzentrierung, direkt als Flüssigpräparation der färberischen Verwendung zugeführt werden.

Gegenstand der vorliegenden Erfindung ist deshalb auch die Verwendung der Farbstoffe (1) zum Färben und insbesondere Bedrucken von hydroxy- und carbonamidgruppenhaltigen Materialien bzw. Verfahren zu deren Anwendung auf diesen Substraten. Bevorzugt kommen die Materialien in Form von Fasermaterialien zur Anwendung, insbesondere in Form von Textilfasern, wie Garnen, Wickelkörpern und Geweben.

Hydroxygruppenhaltige Materialien sind natürliche oder synthetische hydroxygruppenhaltige Materialien, wie beispielsweise Cellulosefasermaterialien, auch in Form von Papier, oder deren Regeneratprodukte und Polyvinylalkohole. Cellulosefasermaterialien sind vorzugsweise Baumwolle, aber auch andere Pflanzenfasern, wie Leinen, Hanf, Jute und Ramiefasern, regenerierte Cellulosefasern sind beispielsweise Zellwolle und Viskosekunstseide.

Carbonamidgruppenhaltige Materialien sind beispielsweise synthetische und natürliche Polyamide und Polyurethane, insbesondere in Form der Fasern, beispielsweise Wolle und andere Tierhaare, Seide, Leder, Polyamid-6,6, Polyamid-6, Polyamid-11 und Polyamid-4.

Die Farbstoffe (1) lassen sich auf den genannten Substraten, insbesondere auf den genannten Fasermaterialien, nach den für wasserlösliche Farbstoffe, insbesondere für faserreaktive Farbstoffe, bekannten Anwendungstechniken applizieren und fixieren. So erhält man mit ihnen auf Cellulosefasern Färbungen in guten Farbausbeuten. Man färbt bei Temperaturen zwischen 40 und 105°C gegebenenfalls bei Temperaturen bis zu 130°C unter Druck, gegebenenfalls in Gegenwart von üblichen Färbereihilfsmitteln, im wäßrigen Bad. Man kann dabei so vorgehen, daß man das Material in das warme Bad einbringt und dieses allmählich auf die gewünschte Färbetemperatur erwärmt und den Färbeprozeß bei dieser Temperatur zu Ende führt. Die das Ausziehen des Farbstoffes beschleunigenden Neutralsalze können dem Bade gewünschtenfalls auch erst nach Erreichen der eigentlichen Färbetemperatur zugesetzt werden.

Nach den Klotzverfahren werden auf Cellulosefasern ebenfalls Färbungen mit ausgezeichneten Farbausbeuten erhalten, wobei durch Verweilen bei Raumtemperatur oder erhöhter Temperatur, beispielsweise bis zu etwa 60°C, durch Dämpfen oder mit Trockenhitze in üblicher Weise fixiert werden kann.

Ebenfalls erhält man nach den üblichen Druckverfahren für Cellulosefasern, - die bevorzugt einphasig durchgeführt werden, beispielsweise durch Bedrucken mit einer Natriumbicarbonat oder ein anderes säurebindendes Mittel und den Farbstoff (1) enthaltenden Druckpaste und durch anschließendes Dämpfen bei 100 bis 103°C, oder zweiphasig durchgeführt werden können, beispielsweise durch Bedrucken mit neutraler oder schwach saurer, den Farbstoff (1) enthaltenden Druckpaste und anschließendes Fixieren entweder durch Hindurchführen der bedruckten Ware durch ein heißes elektrolythaltiges alkalisches Bad oder durch Überklotzen mit einer alkalischen elektrolythaltigen Klotzflotte mit anschließendem Verweilen dieses behandelten Materials oder anschließendem Dämpfen oder anschließender Behandlung mit Trockenhitze, - farbstarke Drucke mit gutem Stand der Konturen und einem klaren Weißfond. Der Ausfall der Drucke ist von wechselnden Fixierbedingungen nur wenig abhängig. Sowohl in der Färberei als auch in der Druckerei sind die mit den Farbstoffen (1) erhaltenen Fixiergrade sehr hoch. Bei der Fixierung mittels Trockenhitze nach den üblichen Thermofixierverfahren verwendet man Heißluft von 120 bis 200°C. Neben dem üblichen Wasserdampf von 101 bis 103°C kann auch überhitzter Dampf und Druckdampf von Temperaturen bis zu 160°C eingesetzt werden.

Die säurebindenden und die Fixierung der Farbstoffe auf den Cellulosefasern bewirkenden Mittel sind beispielsweise wasserlösliche basische Salze der Alkalimetalle und der Erdalkalimetalle von anorganischen oder organischen Säuren, ebenso Verbindungen, die in der Hitze Alkali freizusetzen vermögen. Insbesondere sind die Alkalimetallhydroxide und Alkalimetallsalze von schwachen bis mittelstarken anorganischen oder organischen Säuren zu nennen, wobei von den Alkaliverbindungen vorzugsweise die Natrium- und Kaliumverbindungen gemeint sind. Solche säurebindenden Mittel sind beispielsweise Natriumhydroxid, Kaliumhydroxid, Natriumcarbonat, Natriumbicarbonat, Kaliumcarbonat, Natriumformiat, Natriumdihydrogenphosphat und Dinatriumhydrogenphosphat.

Durch die Behandlung der Farbstoffe (1) mit den säurebindenden Mitteln, gegebenenfalls unter Wärmeeinwirkung, werden die Farbstoffe chemisch an die Cellulosefaser gebunden; insbesondere die Cellulosefärbungen zeigen nach der üblichen Nachbehandlung durch Spülen zur Entfernung von nicht fixierten Farbstoffanteilen ausgezeichnete Naßechtheiten, zumal sich nicht fixierte Farbstoffanteile leicht wegen ihrer guten Kaltwasserlöslichkeit auswaschen lassen.

Die Färbungen auf Polyurethan- und Polyamidfasern werden üblicherweise aus saurem Milieu ausgeführt. So kann man beispielsweise dem Färbebad Essigsäure und/oder Ammoniumsulfat und/oder Essigsäure und Ammoniumacetat oder Natriumacetat zufügen, um den gewünschten pH-Wert zu erhalten. Zur Erreichung einer brauchbaren Egalität der Färbung empfiehlt sich ein Zusatz an üblichen Egalisierhilfsmitteln, wie beispielsweise auf Basis eines Umsetzungsproduktes von Cyanurchlorid mit der dreifach molaren Menge einer Aminobenzolsulfonsäure oder Aminonaphthalinsulfonsäure oder auf Basis eines Umsetzungsproduktes von beispielsweise Stearylamin mit Ethylenoxid. In der Regel wird das zu färbende Material bei einer Temperatur von etwa 40°C in das Bad eingebracht, dort einige Zeit darin bewegt, das Färbebad dann auf den gewünschten schwach sauren, vorzugsweise schwach essigsauren pH-Wert nachgestellt und die eigentliche Färbung bei einer Temperatur zwischen 60 und 98°C durchgeführt. Die Färbungen können aber auch bei Siedetemperatur oder bei Temperaturen bis zu 120°C (unter Druck) ausgeführt werden.

Die mit den erfindungsgemäßen Farbstoffen (1) hergestellten Färbungen und Drucke besitzen eine hohe Farbstärke, außerdem gute Lichtechtheiten und gute Naßechtheiten, wie Wasch-, Walk-, Wasser-, Seewasser-, Überfärbe- und Schweißechtheiten, darüber hinaus eine gute Plissierechtheit, Bügelechtheit und Reibechtheit. Besonders hervorzuheben sind deren alkalische Schweißlichtechtheit und die gute Naßlichtechtheit der mit Trinkwasser benetzten Färbungen.

Die nachstehenden Beispiele dienen zur Erläuterung der Erfindung. Die formelmäßig beschriebenen Verbindungen sind in Form der freien Säure angegeben; im allgemeinen werden sie in Form ihrer Alkalimetallsalze hergestellt und isoliert und in Form ihrer Salze zum Färben verwendet. Ebenso können die in den nachfolgenden Beispielen, insbesondere Tabellenbeispielen, in Form der freien Säure genannten Ausgangsverbindungen als solche oder in Form ihrer Salze, vorzugsweise Alkalimetallsalze, wie Natrium- oder Kaliumsalze, in die Synthese eingesetzt werden.

Die in den Beispielen genannten Teile sind Gewichtsteile, die Prozentangaben stellen Gewichtsprozente dar, sofern nicht anders vermerkt. Gewichtsteile stehen zu Volumenteilen im Verhältnis von Kilogramm zu Liter.

Die für die erfindungsgemäßen Verbindungen angegebenen Absorptionsmaxima (λₘₐₓ) im sichtbaren Bereich wurden anhand ihrer Alkalimetallsalze in wäßriger Lösung ermittelt. In den Tabellenbeispielen sind die λₘₐₓ-Werte bei der Farbtonangabe in Klammern gesetzt; die Wellenlängenangabe bezieht sich auf nm.

Die ¹³C-NMR-Analysen erfolgten in Dimethylsulfoxid und mit Tetramethylsilan als innerem Standard, sofern nicht anders angegeben.

### Beispiel A

a) 584 Teile pulvriges 3-Nitro-benzoylchlorid werden in 15000 Teilen Wasser suspendiert; 157,5 Teile Cyanamid werden hinzugegeben, und es wird mittels 2520 Volumenteilen einer 10%igen wäßrigen Natronlauge ein pH-Wert zwischen 8 und 8,5 eingestellt und während der Umsetzung, die bei 10°C durchgeführt wird, gehalten.
   Die erhaltene Verbindung 3-Nitro-N-cyano-benzamid wird durch Aussalzen mit Natriumchlorid als Natriumsalz aus dem Reaktionsansatz ausgefällt, abfiltriert und getrocknet.
b) In analoger Weise kann, ausgehend vom 4-Nitro-benzoylchlorid, das 4-Nitro-N-cyano-benzamid (als Alkalimetallsalz) hergestellt werden.

### Beispiel B

744 Teile 1-Nitrobenzol-3,5-dicarbonsäurechlorid werden in 15 Teilen Wasser suspendiert; die Suspension wird mit 315 Teilen Cyanamid versetzt, und mittels 10%iger Natronlauge wird ein pH-Wert zwischen 8 und 8,5 eingestellt und während der Umsetzung, die bei einer Temperatur von etwa 10°C durchgeführt wird, gehalten. Anschließend wird die synthetisierte Verbindung 1-Nitrobenzol-3,5-di-(N-cyano-carbonsäureamid) durch Ausfällen bei einem pH-Wert von 1 isoliert. Es stellt eine feste Substanz dar.
Folgende Analysenwerte wurden erhalten:

| | | | | |
|---|---|---|---|---|
| Elementaranalyse: | ber. | C 40,8%, | H 2,1%, | N 29,8%; |
| | gef. | C 35,3%, | H 2,1%, | N 29,5%. |

¹³C-NMR-Analyse (in ppm):
115,7; 125,5; 133,7; 136,7; 147,8; 168,8.

### Beispiel C

Zur Herstellung von 4-Nitro-3-methyl-N-cyano-benzamid verfährt man analog den Angaben des Beispieles A, setzt jedoch als Benzyolchlorid-Ausgangsverbindung das 4-Nitro-3-methyl-benzoylchlorid in äquivalenter Menge ein.
Die Verbindung wird als feste Substanz erhalten. Folgende Analysenwerte wurden ermittelt:

| | | | | |
|---|---|---|---|---|
| Elementaranalyse: | ber. | C 47,5%, | H 2,7%, | N 18,5%; |
| | gef. | C 43,7%, | H 2,8%, | N 17,05%. |

¹³C-NMR-Analyse (in ppm):
19,5; 122,0; 123,7; 126,8; 131,9; 132,3; 142,9; 149,8; 172,7.

### Beispiel D

Zur Herstellung von 3-Nitro-4-methoxy-N-cyano-benzamid verfährt man gemäß den Angaben des Beispieles A, setzt jedoch als Benzoylchlorid-Ausgangsverbindung das 3-Nitro-4-methoxy-benzoylchlorid in äquivalenter Menge ein. Man erhält das Produkt als feste Substanz; sie besitzt folgende physikalische Charakteristika:
¹³C-NMR-Analyse (in ppm):
56,90; 113,4; 122,12; 124,63; 131,3; 134,0; 138,6;
153,47; 172,2.

### Beispiel E

250 Teile 3-Nitro-N-cyano-benzamid (Beispiel A) werden in 2500 Teilen Wasser bei einem pH-Wert von 7,5 suspendiert; 1,5 Teile eines Palladium/Aktivkohle-Katalysators (mit einem Gehalt von 10 % Pd) werden hinzugegeben, und das Produkt wird bei 20 bis 30°C und 50 bar Wasserstoff während zwei Stunden hydriert. Danach wird der Katalysator abfiltriert, das Filtrat mit konzentrierter wäßriger Salzsäure auf einen pH-Wert von 4 gestellt und das ausgefallene 3-Amino-N'-cyano-benzamid abfiltriert und getrocknet.

Es stellt eine feste Substanz dar, die unterhalb von 250°C nicht schmilzt.

### Beispiel F

Zur Herstellung von 4-Amino-N'-cyano-benzamid verfährt man gemäß der Verfahrensweise des Beispieles E, setzt jedoch hierzu als Ausgangsverbindung das isomere 4-Nitro-N-cyanamido-benzamid ein. Die Verbindung wird als feste Substanz erhalten, die unterhalb von 250°C nicht schmilzt.

### Beispiel G

Zur Herstellung von 3-Amino-4-methoxy-N'-cyano-benzamid reduziert man 3-Nitro-4-methoxy-N-cyano-benzamid (Beispiel D) analog den Angaben des Beispieles E. Man erhält eine feste Substanz, die unterhalb von 250°C nicht schmilzt. Folgende Analysenwerte wurden erhalten:

| | | | | |
|---|---|---|---|---|
| Elementaranalyse: | ber. | C 56,6%, | H 4,7%, | N 22,0%; |
| | gef. | C 49,3%, | H 5,2%, | N 19,0%. |

¹³C-NMR-Analyse (in ppm):
55,82; 109,9; 110,6; 113,5; 117,9; 123,3; 137,4; 150,8; 167,4.

### Beispiel H

Zur Herstellung von 4-Amino-3-methyl-N'-cyano-benzamid reduziert man 4-Nitro-3-methyl-N-cyano-benzamid (Beispiel C) analog den Angaben des Beispieles E. Man isoliert das Produkt als feste Substanz.

### Beispiel 1

In eine Lösung von 0°C von 20,5 Teilen 1-Amino-4-(3'-amino-2',4',6'-trimethyl-phenyl)-amino-anthrachinon-2,5'-disulfonsäure in 250 Teilen Wasser werden 7,7 Teile 2,4,6-Trichlor-s-triazin gegeben; während der etwa einstündigem Umsetzung bei 0°C wird mittels wäßrigen Natronlauge ein pH-Wert von 4 gehalten. Danach gibt man 6,6 Teile 4-Amino-N'-cyano-benzamid hinzu, erhöht die Temperatur des Ansatzes auf 10°C und führt die zweite Umsetzung bei einem pH-Wert von 6,5 während zwei Stunden durch.
Die erfindungsgemäße Anthrachinonverbindung wird in üblicher Weise durch Aussalzen mit Natriumchlorid isoliert. Sie besitzt, in Form der freien Säure geschrieben, die Formel 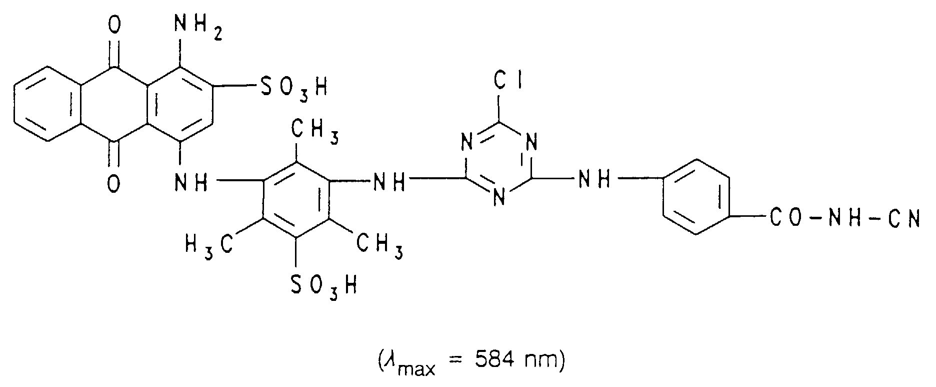 und zeigt sehr gute Farbstoffeigenschaften. Sie liefert gemäß den in der Technik für faserreaktive Farbstoffe üblichen Anwendungsverfahren auf den in der Beschreibung genannten Materialien, insbesondere Cellulosefasermaterialien, wie Baumwolle, farbstarke, echte Färbungen und Drucke in brillanten blauen Tönen mit guten Echtheitseigenschaften.

### Beispiel 2

Man verfährt zur Herstellung eines erfindungsgemäßen Anthrachinonfarbstoffes analog den Angaben des Beispieles 1, setzt jedoch anstelle der dort angegebenen Anthrachinon-Ausgangsverbindung die äquivalente Menge an 1-Amino-4-(2'-methyl-3'-amino-phenyl)-amino-anthrachinon-2,5'-disulfonsäure ein. Man isoliert den erfindungsgemäßen Anthrachinonfarbstoff der Formel 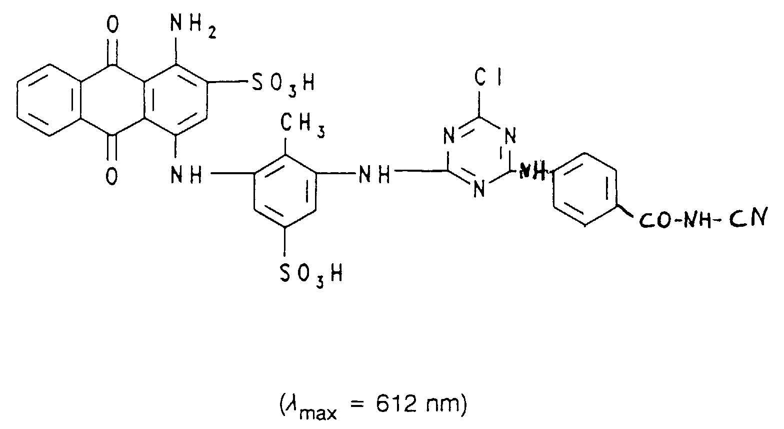 als Natriumsalz. Man erhält mit ihm auf den in der Beschreibung genannten Materialien, insbesondere Cellulosefasermaterialien, nach den in der Technik für faserreaktive Farbstoffe üblichen Applikations- und Fixierverfahren farbstarke, echte, brillante blaue Färbungen und Drucke.

### Beispiel 3

Zu einer Lösung von 51 Teilen 1-Amino-4-(3'-amino-2',4',6-trimethyl-phenyl)-amino-anthrachinon-2,4'-disulfonsäure in 350 Teilen Wasser von 0°C werden langsam unter Einhaltung dieser Temperatur und einem pH-Wert von 5 10 Volumenteilen Cyanurfluorid gegeben; während der Umsetzung wird der pH-Wert von 5 mittels wäßriger 2n-Natronlauge gehalten. Danach gibt man 16 Teile 3-Amino-N'-cyano-benzamid hinzu, erhöht die Temperatur des Ansatzes auf 5 bis 10°C und führt die Umsetzung bei einem pH-Wert von 6 während 3 Stunden durch.
Der erfindungsgemäße Anthrachinonfarbstoff wird mittels Natriumchlorid ausgesalzen und als Alkalimetallsalz in Form eines blauen elektrolytsalzhaltigen (natriumchloridhaltigen) blauen Pulvers isoliert. Er besitzt, in Form der freien Säure geschrieben, die Formel 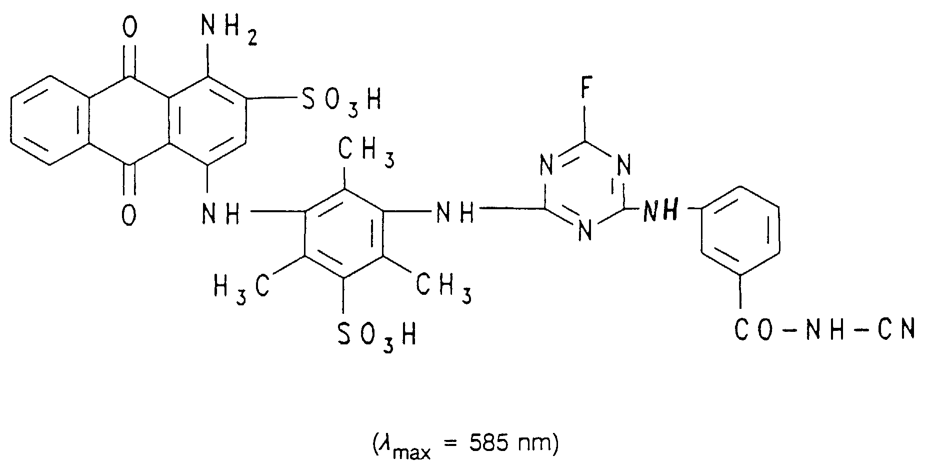 zeigt sehr gute Farbstoffeigenschaften und färbt beispielsweise Baumwolle nach den in der Technik für faserreaktive Farbstoffe üblichen Anwendungsverfahren in echten, brillanten blauen Tönen.

### Beispiele 4 bis 16

In den nachstehenden Tabellenbeispielen sind weitere erfindungsgemäße Anthrachinonfarbstoffe entsprechend einer allgemeinen Formel (A) 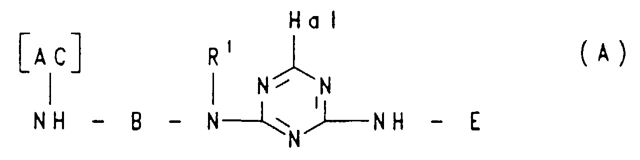 mit Hilfe ihrer Komponenten (dem Anthrachinonrest AC entsprechend der allgemeinen Formel (a) 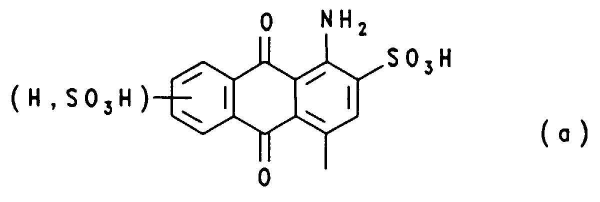 dem bivalenten Rest B, dem Halogenatom Chlor oder Fluor und dem Cyanobenzamid-Rest E) beschrieben. Sie lassen sich in erfindungsgemäßer Weise, beispielsweise analog einem der oben angegebenen Beispiele unter Einsatz einer Anthrachinonverbindung entsprechend der allgemeinen Formel AC-NH-B-NHR¹, Cyanurchlorid oder Cyanurfluorid und einer Cyanamidocarbonyl-anilin-Verbindung entsprechend der allgemeinen Formel H₂N-E herstellen. Diese neuen Anthrachinonfarbstoffe besitzen sehr gute faserreaktive Farbstoffeigenschaften und färben die in der Beschreibung genannten Materialien, insbesondere Cellulosefasermaterialien, wie Baumwolle, in dem in dem jeweiligen Tabellenbeispiel (hier für Baumwolle) angegebenen Farbton in hoher Farbstärke und mit guten Echtheiten.

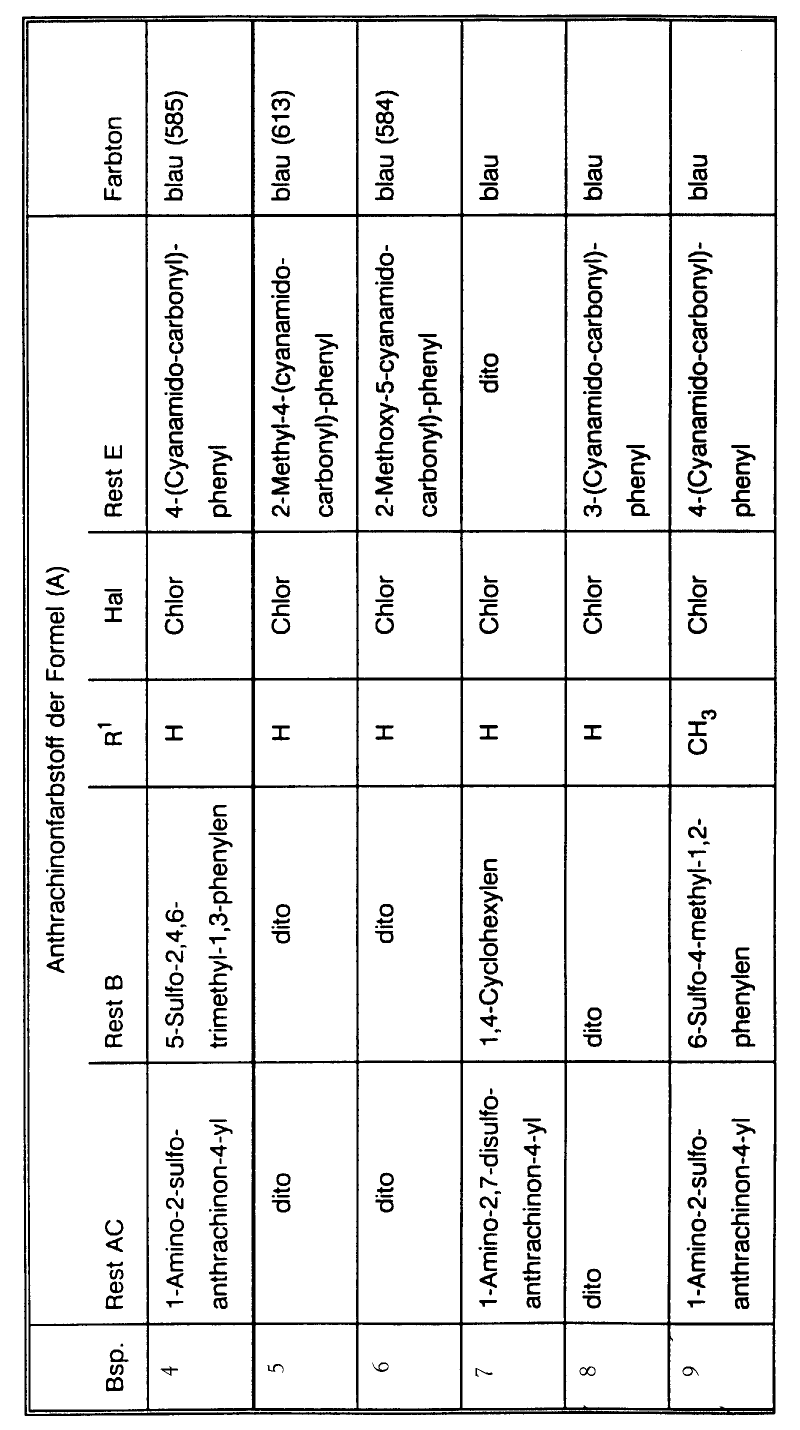

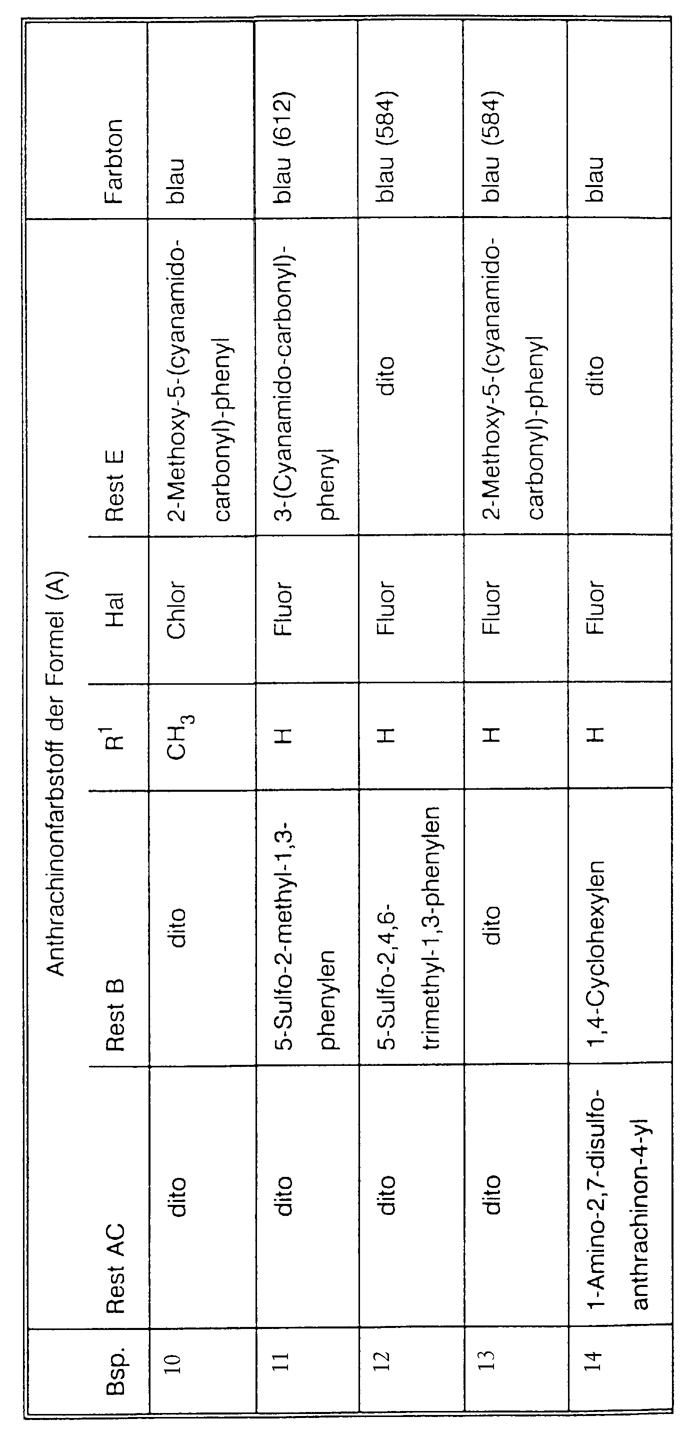

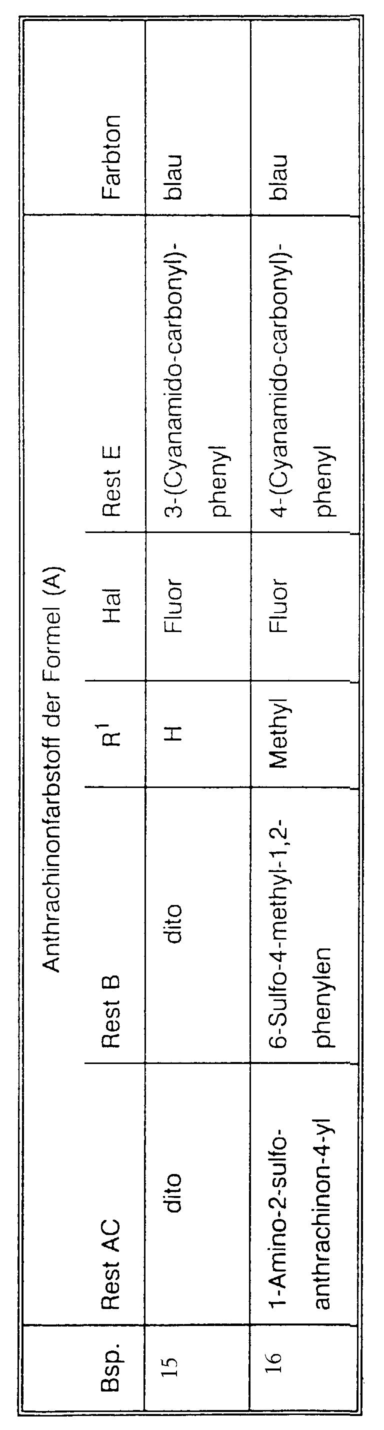

## Patentansprüche

1. Anthrachinonverbindung entsprechend der allgemeinen Formel (1) 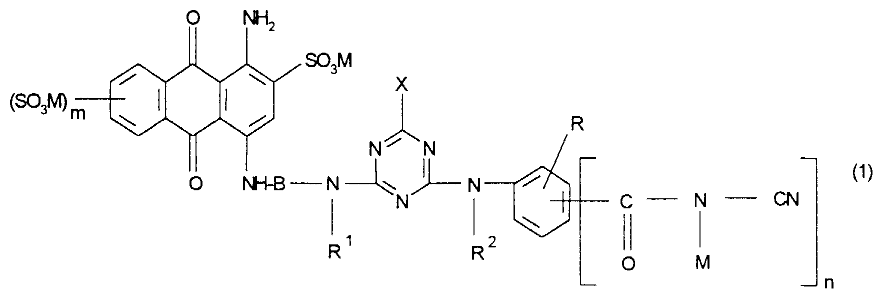 in welcher bedeuten:
m ist die Zahl Null, 1 oder 2 (wobei im Falle von m gleich Null diese Gruppe für Wasserstoff steht);
B ist Phenylen, das durch 1 bis 2 Sulfogruppen und 1 bis 3 Alkylreste von 1 bis 4 C-Atomen substituiert ist; oder ist Phenylen-alkylen oder Alkylen-phenylen, wobei deren Alkylenreste solche von 1 bis 4 C-Atomen sind und die Phenylenreste durch 1, 2 oder 3 Substituenten aus der Gruppe Alkyl von 1 bis 4 C-Atomen und Sulfo substituiert sind, oder ist (C₅-C₈)-Cycloalkylen oder Alkylen-(C₅-C₈)-cycloalkylen oder (C₅-C₈)-Cycloalkylen-alkylen oder Alkylen-(C₅-C₈)-cycloalkylen-alkylen, wobei die Cycloalkylen-Reste noch durch 1 oder 2 Methylgruppen substituiert sein können und die Alkylenreste solche von 1 bis 4 C-Atomen sind, oder ist ein Rest der allgemeinen Formel -phen-D-phen- , in welcher jedes phen eine zueinander gleiche oder voneinander verschiedene Bedeutung besitzt und einen Phenylenrest bedeutet, der durch 1 oder 2 Substituenten aus der Gruppe Sulfo, Alkyl C₁₋₄ und Alkoxy C₁₋₄ substituiert ist, und D eine direkte Bindung oder eine Gruppe der Formel -NH-, -O-, -SO₂-, -CO-NH-, -NH-CO-, -SO₂-NH-, -NH-SO₂- und -SO₂-NH-SO₂- bedeutet,
R¹ ist Wasserstoff oder Alkyl von 1 bis 4 C-Atomen;
R² ist Wasserstoff oder Alkyl von 1 bis 4 C-Atomen;
X ist Fluor oder Chlor;
R ist Wasserstoff, Alkyl von 1 bis 4 C-Atomen oder Alkoxy von 1 bis 4 C-Atomen;
n ist die Zahl von 1 oder 2; und
M ist Wasserstoff oder ein Alkalimetall oder das Moläquivalent eines Erdalkalimetalls.

2. Antrachinonverbindung nach Anspruch 1 der allgemeinen Formel (3a) 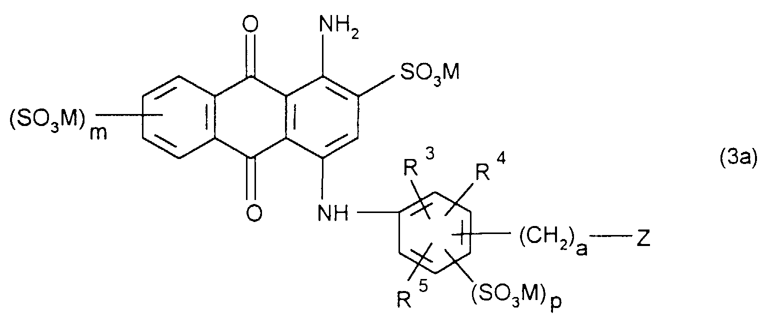 in welcher bedeuten:
M ist Wasserstoff oder ein Alkalimetall oder das Moläquivalent eines Erdalkalimetalls;
m ist die Zahl Null, 1 oder 2 (wobei im Falle von m gleich Null diese Gruppe für Wasserstoff steht);
R³ ist Alkyl von 1 bis 4 C-Atomen;
R⁴ ist Wasserstoff oder Alkyl von 1 bis 4 C-Atomen;
R⁵ ist Wasserstoff oder Alkyl von 1 bis 4 C-Atomen;
p ist die Zahl 1 oder 2;
a ist die Zahl Null oder 1;
Z ist ein Rest der allgemeinen Formel (4) 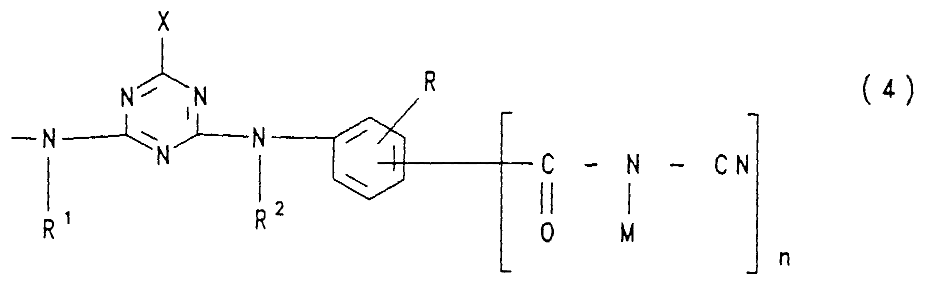
in welcher R¹, R², R, M, n und X die in Anspruch 1 genannten Bedeutungen haben.

3. Anthrachinonverbindung nach Anspruch 1 der allgemeinen Formel (3b) 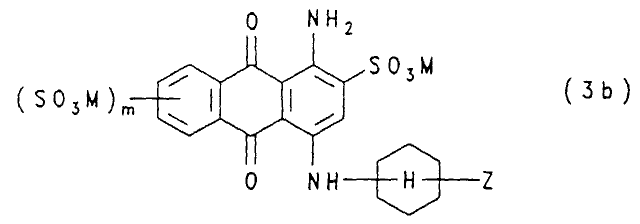 in welcher
M Wasserstoff oder ein Alkalimetall oder das Moläquivalent eines Erdalkalimetalls ist,
m für die Zahl Null, 1 oder 2 steht (wobei im Falle von m gleich Null diese Gruppe Wasserstoff bedeutet) und
Z ein Rest der allgemeinen Formel (4) 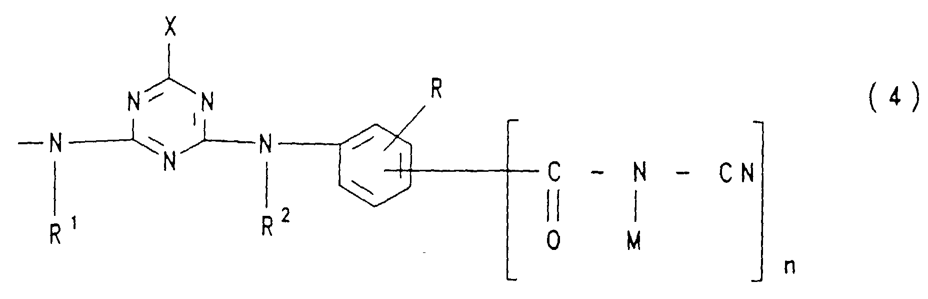 ist, in welcher R¹, R²,R, M, n und X die in Anspruch 1 genannten Bedeutungen haben.

4. Anthrachinonverbindung nach Anspruch 1 entsprechend der allgemeinen Formel (5) 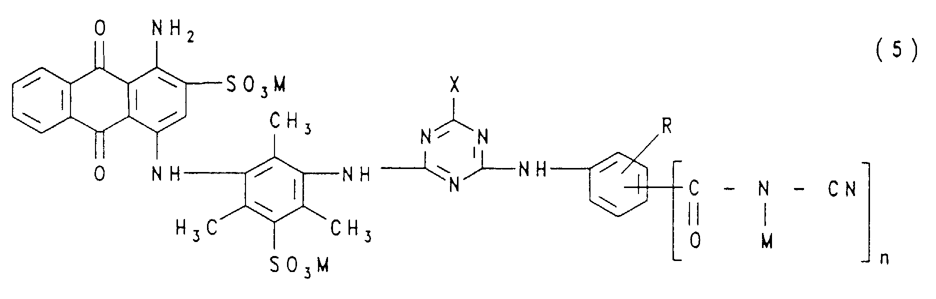 in welcher X, M, R und n die in Anspruch 1 genannten Bedeutungen haben.

5. Anthrachinonverbindung nach mindestens einem der Ansprüche 1 bis 4, dadurch gekennzeichnet, daß n die Zahl 1 ist.

6. Anthrachinonverbindung nach Anspruch 1 der Formel 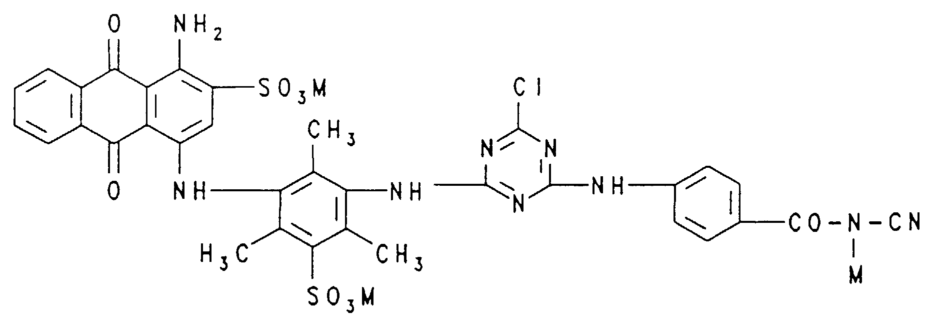 in welcher M die in Anspruch 1 genannte Bedeutung besitzt.

7. Anthrachinonverbindung nach Anspruch 1 der Formel 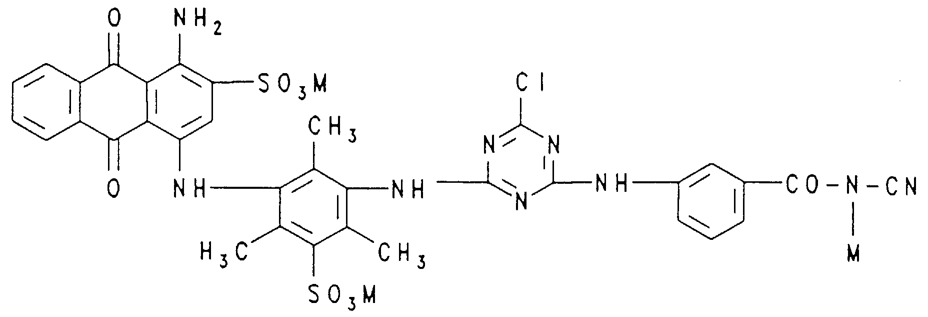 in welcher M die in Anspruch 1 genannte Bedeutung besitzt.

8. Anthrachinonverbindung nach Anspruch 1 der Formel 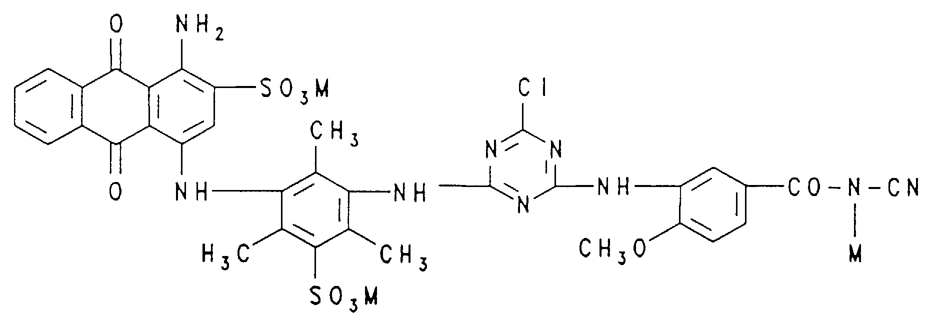 in welcher M die in Anspruch 1 genannte Bedeutung besitzt.

9. Anthrachinonverbindung nach Anspruch 1 der Formel 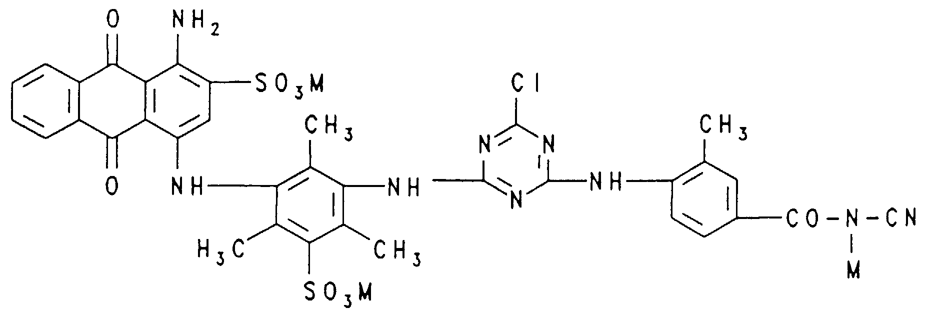 in welcher M die in Anspruch 1 genannte Bedeutung besitzt.

10. Verfahren zur Herstellung einer Verbindung der in Anspruch 1 genannten und definierten allgemeinen Formel (1), dadurch gekennzeichnet, daß man eine Halogen-s-triazinverbindung der allgemeinen Formel (6) 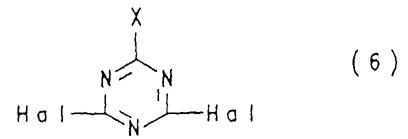 in welcher X die in Anspruch 1 genannte Bedeutung besitzt und Hal für Halogen steht, mit einer aminogruppenhaltigen Anthrachinonverbindung der allgemeinen Formel 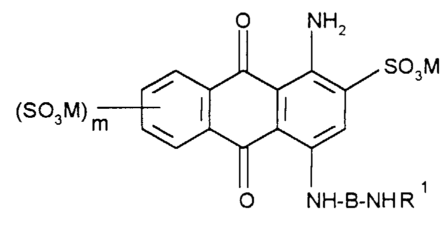 und mit einer Aminoverbindung der allgemeinen Formel (7) 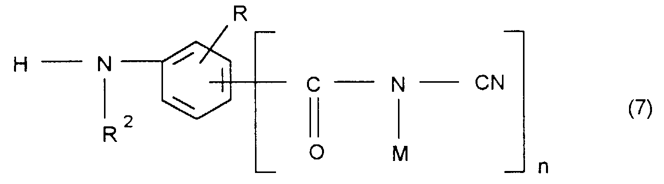 in welcher R², R, M und n die in Anspruch 1 genannten Bedeutungen haben, in beliebiger Reihenfolge miteinander umsetzt.

11. Verwendung eines Anthrachinonfarbstoffes von mindestens einem der Ansprüche 1 bis 9 oder eines nach Anspruch 10 hergestellten Anthrachinonfarbstoffes zum Färben und Bedrucken von hydroxy- und/oder carbonamidgruppenhaltigem Material, insbesondere Fasermaterial.

12. Verfahren zum Färben und Bedrucken von hydroxy- und/oder carbonamidgruppenhaltigem Material, vorzugsweise Fasermaterial, bei welchem man einen Farbstoff auf das Material aufbringt und den Farbstoff auf dem Material mittels Wärme oder mit Hilfe eines alkalisch wirkenden Mittels oder mittels Wärme und mit Hilfe eines alkalisch wirkenden Mittels fixiert, dadurch gekennzeichnet, daß man als Farbstoff einen Anthrachinonfarbstoff von mindestens einem der Ansprüche 1 bis 9 oder einen nach Anspruch 10 hergestellten Anthrachinonfarbstoff verwendet.

13. Verbindung entsprechend der allgemeinen Formel 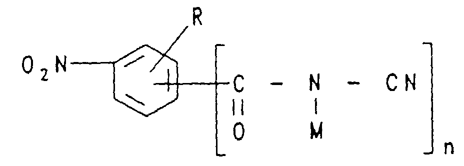 in welcher
R Alkyl von 1 bis 4 C-Atomen oder Alkoxy von 1 bis 4 C-Atomen ist,
M Wasserstoff oder ein Alkalimetall oder das Moläquivalent eines Erdalkalimetalls ist und
n für die Zahl 1 oder 2 steht.

14. Verbindung entsprechend der allgemeinen Formel (7) 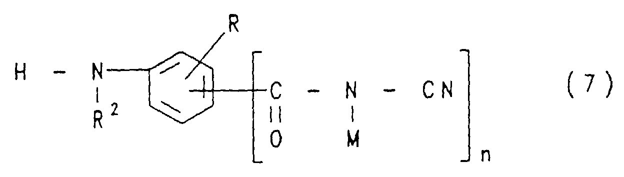 in welcher
R² Wasserstoff oder Alkyl von 1 bis 4 C-Atomen ist,
R Alkyl von 1 bis 4 C-Atomen oder Alkoxy von 1 bis 4 C-Atomen ist,
M Wasserstoff oder ein Alkalimetall oder das Moläquivalent eines Erdalkalimetalls ist und
n für die Zahl 1 oder 2 steht.

15. Verfahren zur Herstellung einer Verbindung von Anspruch 13 oder Anspruch 14, dadurch gekennzeichnet, daß man eine Verbindung der allgemeinen Formel (11) 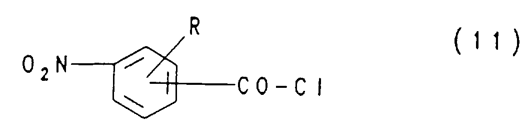 in welcher R Alkyl von 1 bis 4 C-Atomen oder Alkoxy von 1 bis 4 C-Atomen ist, mit Cyanamid oder einem Salz des Cyanamids in wäßriger Lösung oder Suspension bei einer Temperatur zwischen 0 und 20°C und bei einem pH-Wert zwischen 7 und 11 umsetzt und im Falle der Herstellung einer Aminoverbindung von Anspruch 14 die erhaltene Nitroverbindung der allgemeinen Formel (10) 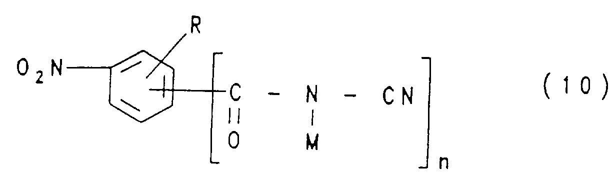 in welcher R, M und n die Anspruch 13 genannten Bedeutungen haben, zur Aminoverbindung der allgemeinen Formel (7) mit R² gleich Wasserstoff reduziert und diese gegebenenfalls mit einem Alkylierungsmittel in eine Verbindung der allgemeinen Formel (7) mit R² gleich Alkyl von 1 bis 4 C-Atomen überführt.

## Claims

1. An anthraquinone compound conforming to the formula 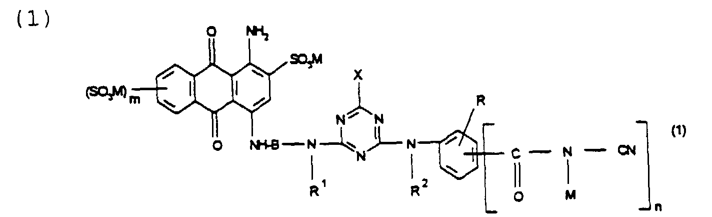 where
m is zero, 1 or 2 (when zero, the group in question is hydrogen),
B is phenylene which is substituted by 1 to 2 sulfo groups and 1 to 3 alkyl radicals of 1to 4 carbon atoms; or is phenylenealkylene or alkylene-phenylene, wherein the alkylene radicals have 1 to 4 carbon atoms and the phenylene radicals are substituted by 1, 2 or 3 substituents from the group consisting of alkyl of 1 to 4 carbon atoms and sulfo, or is (C₅-C₈)cycloalkylene or alkylene(C₅-C₈)cyclo-alkylene or (C₅-C₈)cycloalkylenealkylene or alkylene(C₅-C₈)-cycloalkylenealkylene, wherein the cycloalkylene radicals may additionally be substituted by 1 or 2 methyl groups and the alkylene radicals are those of 1 to 4 carbon atoms, or is a radical of the formula -phen-D-phen-, in which each phen is identical to or different from the other, is phenylene which is substituted by 1 or 2 substituents from the group consisting of sulfo, alkylC₁₋₄ and alkoxyC₁₋₄, and D is a direct bond or a group of the formula -NH-, -O-, -SO₂-, -CO-NH-, -NH-CO-, -SO₂-NH-, -NH-SO₂- and -SO₂ -NH-SO₂-,
R¹ is hydrogen or alkyl of 1 to 4 carbon atoms,
R² is hydrogen or alkyl of 1 to 4 carbon atoms,
X is fluorine or chlorine,
R is hydrogen, alkyl of 1 to 4 carbon atoms, or alkoxy of 1 to 4 carbon atoms,
n is 1 or 2, and
M is hydrogen or an alkali metal or the mole equivalent of an alkaline earth metal.

2. An anthraquinone compound as claimed in claim 1 of the formula (3a) 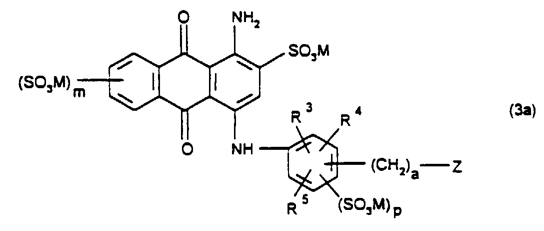 where
M is hydrogen or an alkali metal or the mole equivalent of an alkaline earth metal,
m is zero, 1 or 2 (when zero, the group in question is hydrogen),
R³ is alkyl of 1 to 4 carbon atoms,
R⁴ is hydrogen or alkyl of 1to 4 carbon atoms,
R⁵ is hydrogen or alkyl of 1 to 4 carbon atoms,
p is zero, 1 or 2,
a is zero or 1,
Z is a radical of the formula (4) 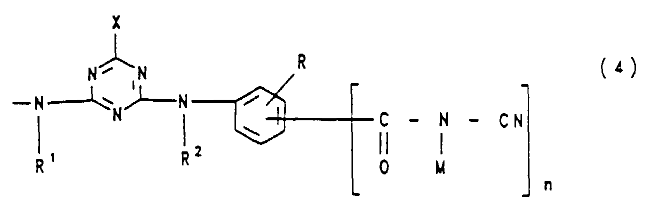
where R¹, R², R, M, n and X are each as defined in claim 1.

3. An anthraquinone compound as claimed in claim 1 of the formula (3b) 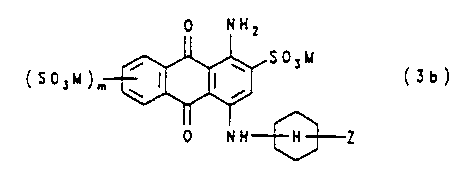 where
M is hydrogen or an alkali metal or the mole equivalent of an alkaline earth metal,
m is zero, 1 or 2 (when zero, the group in question is hydrogen) and
Z is a radical of the formula (4) 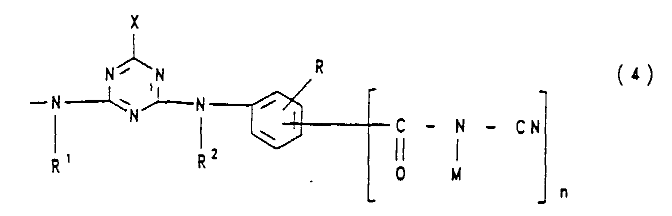
where R¹, R², R, M, n and X are each as defined in claim 1.

4. An anthraquinone compound as claimed in claim 1 conforming to the formula (5) 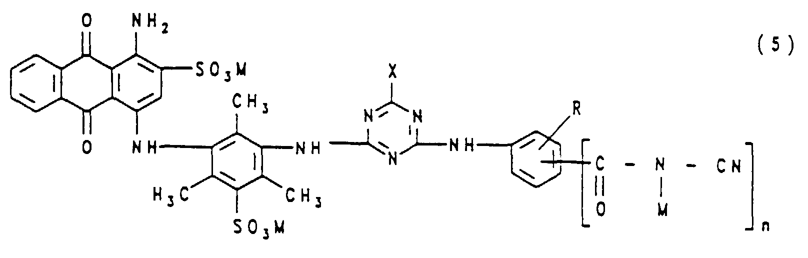 where X, M, R and n are each as defined in claim 1.

5. An anthraquinone compound as claimed in at least one of claims 1 to 4, wherein n is 1.

6. An anthraquinone compound as claimed in claim 1 of the formula 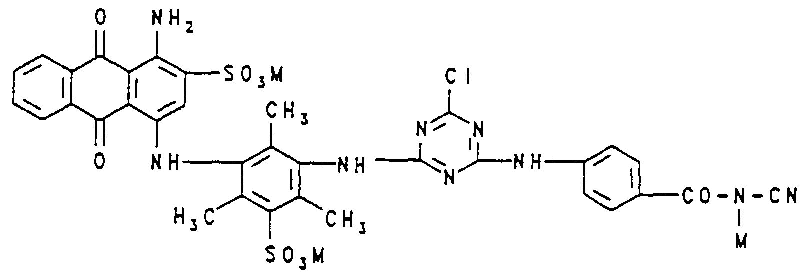 where M is as defined in claim 1.

7. An anthraquinone compound as claimed in claim 1 of the formula 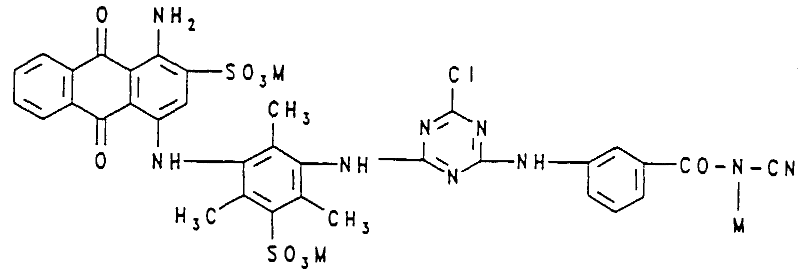 where M is as defined in claim 1.

8. An anthraquinone compound as claimed in claim 1 of the formula 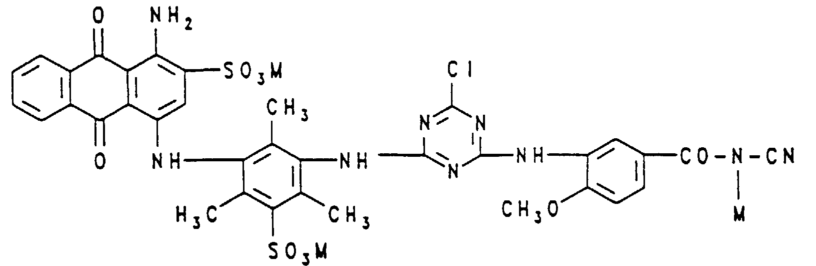 where M is as defined in claim 1.

9. An anthraquinone compound as claimed in claim 1 of the formula 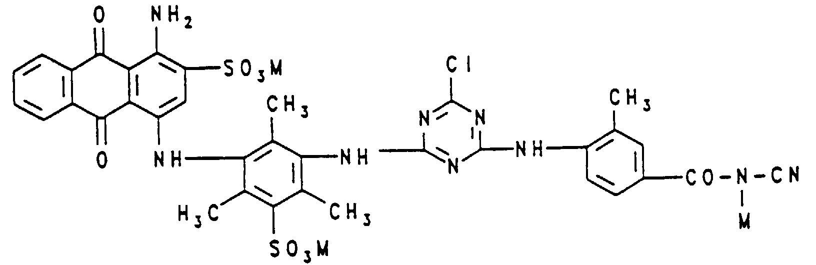 where M is as defined in claim 1.

10. A process for preparing a compound of the formula (1) mentioned and defined in claim 1, which comprises reacting a halo-s-triazine compound of the formula (6) 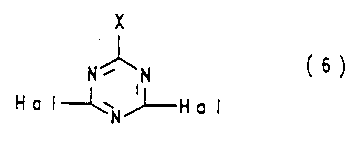 where X is as defined in claim 1 and Hal is halogen, with an amino-containing anthraquinone compound of the formula 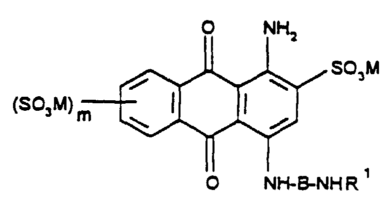 and with an amino compound of the formula (7) 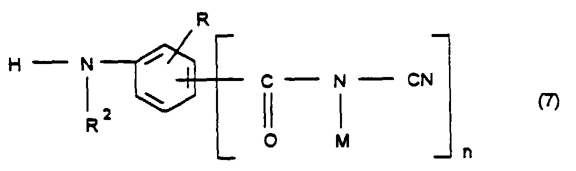 where R², R, M and n are each as defined in claim 1, in any desired order.

11. The use of an anthraquinone dye of at least one of claims 1 to 9 or of an anthraquinone dye prepared as claimed in claim 10, for dyeing and printing hydroxyl- and/or carboxamide-containing material, in particular fiber material.

12. A process for dyeing and printing hydroxyl- and/or carboxamido-containing material, preferably fiber material, by applying a dye to the material and fixing it thereon by means of heat, or with the aid of an alkali, or by means of heat and with the aid of an alkali, which comprises using a dye that is an anthraquinone dye of at least one of claims 1to 9 or an anthraquinone dye prepared as claimed in claim 10.

13. A compound conforming to the formula 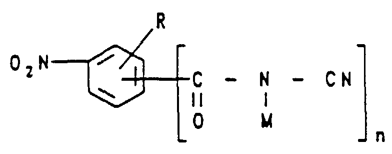 where
R is alkyl of 1 to 4 carbon atoms or alkoxy of 1 to 4 carbon atoms,
M is hydrogen or an alkali metal or the mole equivalent of an alkaline earth metal and
n is 1 or 2.

14. A compound conforming to the formula (7) 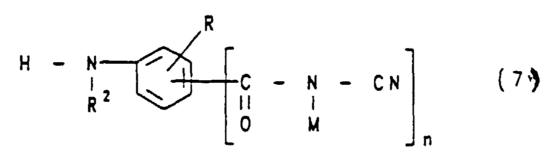 where
R² is hydrogen or alkyl of 1 to 4 carbon atoms,
R is alkyl of 1 to 4 carbon atoms or alkoxy of 1 to 4 carbon atoms,
M is hydrogen or an alkali metal or the mole equivalent of an alkaline earth metal and
n is 1 or 2.

15. A process for preparing a compound as claimed in claim 13 or 14, which comprises reacting a compound of the formula (11) 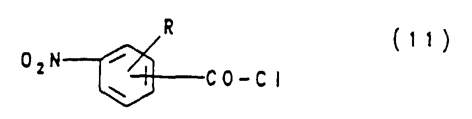 where R is alkyl of 1 to 4 carbon atoms or alkoxy of 1 to 4 carbon atoms, with cyanamide or a salt of cyanamide in aqueous solution or suspension at a temperature between 0 and 20°C and at a pH between 7 and 11 and in the case of the preparation of an amino compound of claim 14 reducing the resulting nitro compound of the formula (10) 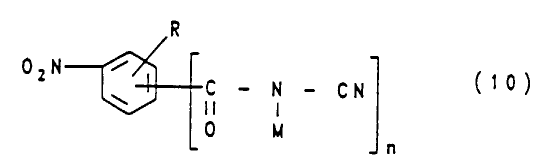 where R, M and n are each as defined in claim 13, to the amino compound of the formula (7) where R² is hydrogen and optionally converting it with an alkylating agent into a compound of the formula (7) where R² is alkyl of 1 to 4 carbon atoms.

## Revendications

1. Composé anthraquinone correspondant à la formule générale (1) 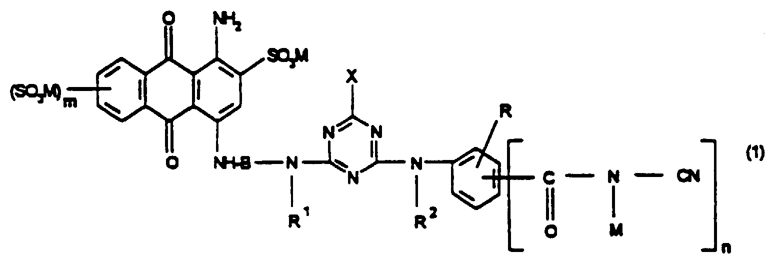 dans laquelle:
m est le nombre zéro, 1 ou 2 (ce groupe désignant un hydrogène dans le cas où m est égal à zéro);
B est un phénylène qui est substitué par 1 à 2 groupes sulfo et 1 à 3 radicaux alkyle ayant de 1 à 4 atomes de carbone; ou est un phénylène-alkylène ou alkylène-phenylène, dont les radicaux alkylène ont de 1 à 4 atomes de carbone et les radicaux phénylène sont substitués par 1, 2 ou 3 substituants choisis parmi le groupe constitué d'un alkyle ayant de 1 à 4 atomes de carbone et d'un sulfo, ou est un cycloalkylène en C₅-C₈, un alkylène-(C₅-C₈)-cycloalkylène, un (C₅-C₈)-cycloalkylène-alkylène ou un alkylène-(C₅-C₈)-cycloalkylène-alkylène, les radicaux cycloalkylène pouvant encore être substitués par 1 ou 2 groupes méthyle et les radicaux alkylène ayant de 1 à 4 atomes de carbone, ou est un radical de formule générale -phén-D-phén-, dans laquelle chaque phén est identique ou différent et représente un radical phénylène, qui est substitué par 1 ou 2 substituants choisis parmi le groupe constitué d'un sulfo, d'un alkyle en C₁₋₄, et d'un alcoxy en C₁₋₄, et D représente une liaison directe ou un groupe de formule -NH-, O-, -SO₂-, -CO-NH-, -NH-CO-, -SO₂-NH-, -NH-SO₂- et SO₂-NH-SO₂,
R¹ est un hydrogène ou un alkyle ayant de 1 à 4 atomes de carbone;
R² est un hydrogène ou un alkyle ayant de 1 à 4 atomes de carbone;
X est un fluor ou un chlore;
R est un hydrogène, un alkyle ayant de 1 à 4 atomes de carbone ou un alcoxy ayant de 1 à 4 atomes de carbone;
n est le nombre 1 ou 2, et
M est un hydrogène, un métal alcalin ou l'équivalent molaire d'un métal alcalino-terreux.

2. Composé anthraquinone selon la revendication 1, de formule générale (3a) 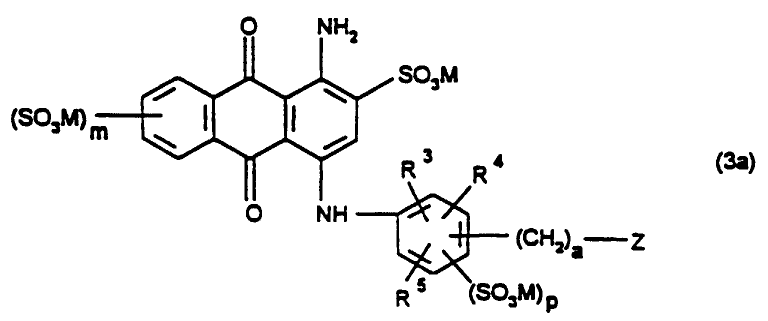 dans laquelle:
M est un hydrogène, un métal alcalin ou l'équivalent molaire d'un métal alcalino-terreux;
m est le nombre zéro, 1 ou 2 (ce groupe désignant un hydrogène dans le cas où m vaut zéro);
R³ est un alkyle ayant de 1 à 4 atomes de carbone;
R⁴ est un hydrogène ou un alkyle ayant de 1 à 4 atomes de carbone;
R⁵ est un hydrogène ou un alkyle ayant de 1 à 4 atomes de carbone;
p est le nombre 1 ou 2;
a est le nombre zéro ou 1;
Z est un radical de formule générale (4) 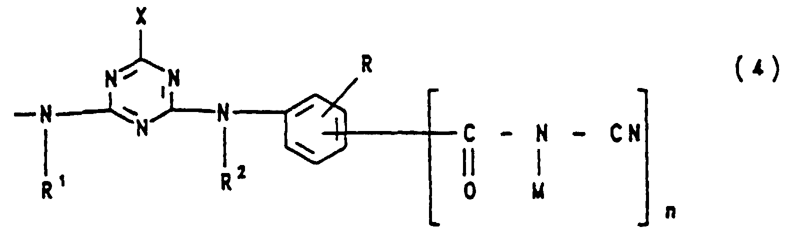
dans laquelle R¹, R², R, M, n et X sont comme définis à la revendication 1.

3. Composé anthraquinone selon la revendication 1, de formule générale (3b) 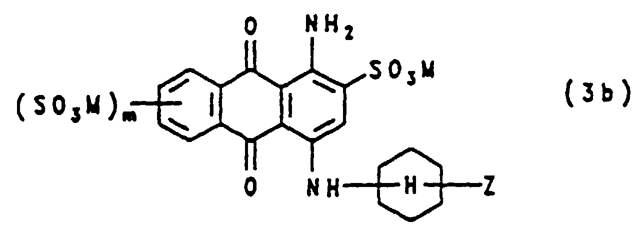 dans laquelle
M est un hydrogène, un métal alcalin ou l'équivalent molaire d'un métal alcalino-terreux;
m désigne le nombre zéro, 1 ou 2 (ce groupe désignant un hydrogène dans le cas où m vaut zéro); et
Z est un radical de formule générale (4) 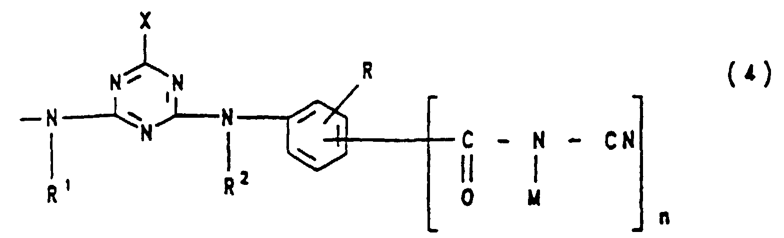
dans laquelle R¹, R², R, M, n et X sont comme définis à la revendication 1.

4. Composé anthraquinone selon la revendication 1, correspondant à la formule générale (5) 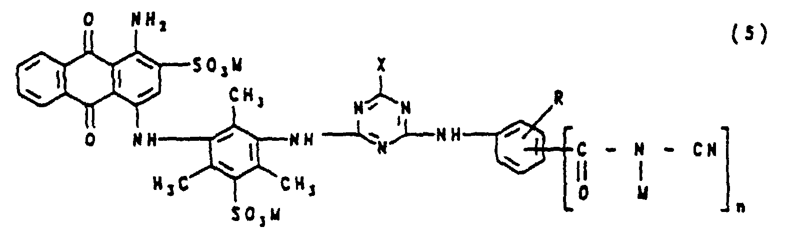 dans laquelle X, M, R et n sont comme définis à la revendication 1.

5. Composé anthraquinone selon l'une au moins des revendications 1 à 4, caractérisé en ce que n est le nombre 1.

6. Composé anthraquinone selon la revendication 1, de formule 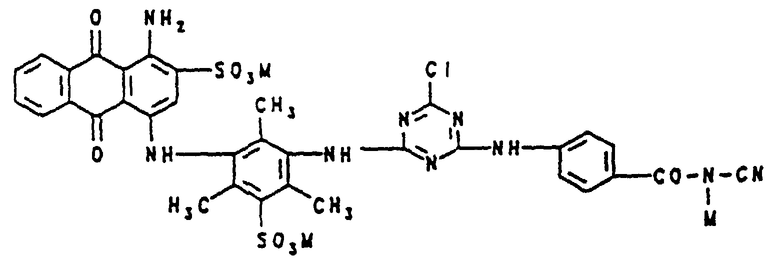 dans laquelle M est comme défini à la revendication 1.

7. Composé anthraquinone selon la revendication 1, de formule 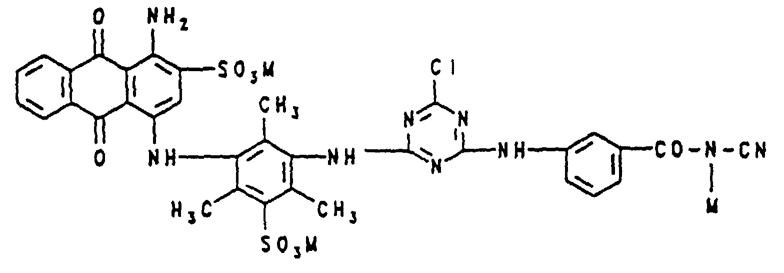 dans laquelle M est comme défini à la revendication 1.

8. Composé anthraquinone selon la revendication 1, de formule 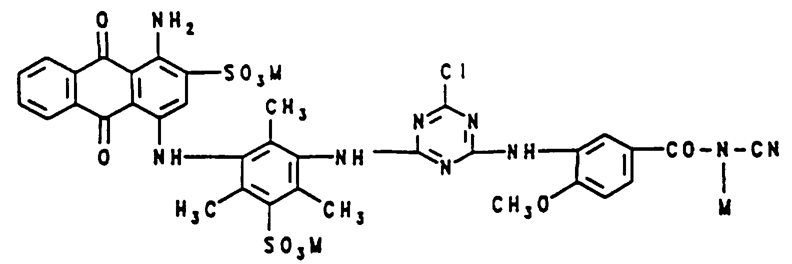 dans laquelle M est comme défini à la revendication 1.

9. Composé anthraquinone selon la revendication 1, de formule 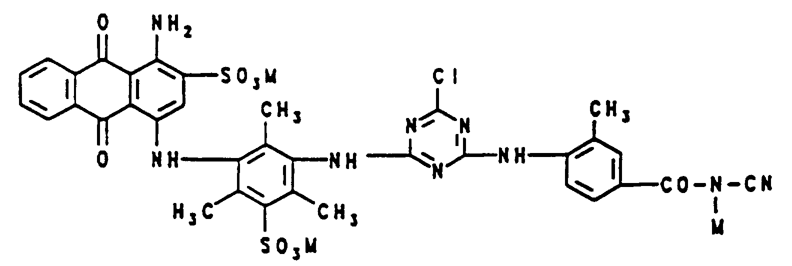 dans laquelle M est comme défini à la revendication 1.

10. Procédé de préparation d'un composé de formule générale (1) mentionnée et définie à la revendication 1, caractérisé en ce que l'on fait réagir dans un ordre quelconque un composé halogéno-s-triazine de formule générale (6) 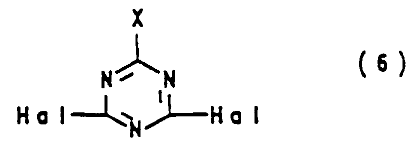 dans laquelle X est comme défini à la revendication 1 et Hal désigne un halogène, avec un composé anthraquinone contenant des groupes amino de formule générale 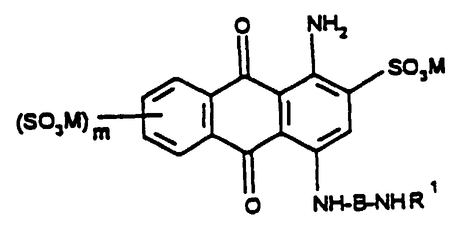 et avec un composé amino de formule générale (7) 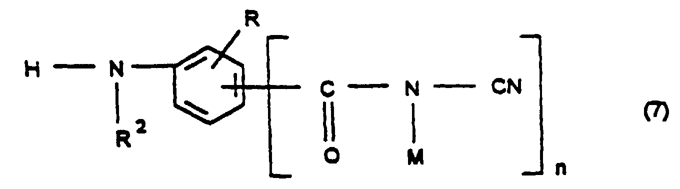 dans laquelle R², R, M et n sont comme définis à la revendication 1.

11. Utilisation d'un colorant anthraquinone selon l'une au moins des revendications 1 à 9 ou d'un colorant anthraquinone préparé selon la revendication 10, en vue de la coloration et l'impression d'un matériau contenant des groupes hydroxy et/ou carbonamide, en particulier d'un matériau fibreux.

12. Procédé de coloration et d'impression d'un matériau contenant des groupes hydroxy et/ou carbonamide, de préférence d'un matériau fibreux, dans lequel on applique un colorant sur le matériau et on fixe le colorant sur le matériau au moyen de chaleur ou à l'aide d'un agent à activité alcaline, ou au moyen de chaleur et à l'aide d'un agent à activité alcaline, caractérisé en ce que l'on emploie, en tant que colorant, un colorant anthraquinone selon l'une au moins des revendications 1 à 9 ou un colorant anthraquinone préparé selon la revendication 10.

13. Composé correspondant à la formule générale 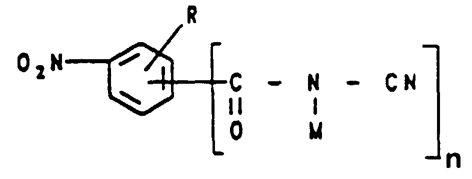 dans laquelle
R est un alkyle ayant de 1 à 4 atomes de carbone ou un alcoxy ayant de 1 à 4 atomes de carbone,
M est un hydrogène, un métal alcalin ou l'équivalent molaire d'un métal alcalino-terreux, et
n désigne le nombre 1 ou 2.

14. Composé correspondant à la formule générale (7) 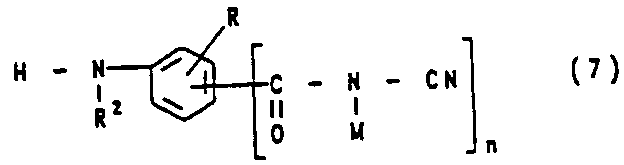 dans laquelle
R² est un hydrogène ou un alkyle ayant de 1 à 4 atomes de carbone,
R est un alkyle ayant de 1 à 4 atomes de carbone ou un alcoxy ayant de 1 à 4 atomes de carbone,
M est un hydrogène, un métal alcalin ou l'équivalent molaire d'un métal alcalino-terreux, et
n désigne le nombre 1 ou 2.

15. Procédé de préparation d'un composé selon la revendication 13 ou la revendication 14, caractérisé en ce que l'on fait réagir un composé de formule générale (11) 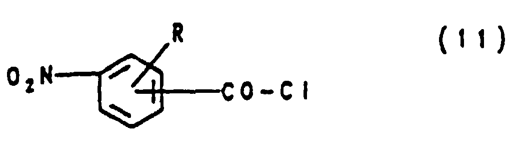 dans laquelle R est un alkyle ayant de 1 à 4 atomes de carbone ou un alcoxy ayant de 1 à 4 atomes de carbone, avec du cyanamide ou un sel de cyanamide en solution ou suspension aqueuse à une température comprise entre 0 et 20°C et à un pH compris entre 7 et 11, et dans le cas de la préparation d'un composé amino selon la revendication 14, on réduit le composé nitro obtenu de formule générale (10) 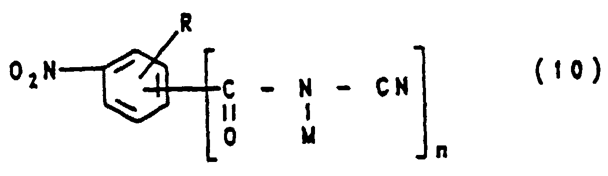 dans laquelle R, M et n sont comme définis à la revendication 13, en composé amino de formule générale (7) où R² est un hydrogène, et on transforme éventuellement celui-ci par un agent d'alkylation en un composé de formule générale (7) où R² est un alkyle ayant de 1 à 4 atomes de carbone.
